# EUROPEAN PATENT SPECIFICATION

(11) **EP 3 976 145 B1**
(45) Date of publication and mention of the grant of the patent: **15.01.2025**
(21) Application number: 19731881.9
(22) Date of filing: 31.05.2019
(51) Int. Cl.: A61M 15/00, A61B 5/00, A61K 9/00

(54) **AN INHALER FOR ELECTRONICALLY SUPERVISED PARENTERAL ADMINISTRATION OF A PHARMACEUTICAL COMPOSITION**
INHALATOR ZUR ELEKTRONISCH ÜBERWACHTEN PARENTERALEN VERABREICHUNG EINER PHARMAZEUTISCHEN ZUSAMMENSETZUNG
INHALATEUR PERMETTANT UNE ADMINISTRATION PARENTÉRALE SURVEILLÉE ÉLECTRONIQUEMENT D'UNE COMPOSITION PHARMACEUTIQUE

(43) Date of publication of application: 06.04.2022
(73) Proprietor: CELON PHARMA S.A., 05-092 Kielpin (PL)
(72) Inventor: WIECZOREK, Maciej, 05-092 Kielpin (PL); WIECZOREK, Artur, 05-092 Kielpin (PL); TRATKIEWICZ, Ewa, 05-092 Kielpin (PL); MAJSTRUK, Maciej, 05-092 Kielpin (PL)
(74) Representative: Markieta, Jaroslaw Franciszek
(86) International application number: PCT/EP2019/064244
(87) International publication number: WO 2020/239244

(56) References cited:
- EP-A1- 3 111 978
- WO-A1-2014/020155
- WO-A1-2014/152196
- WO-A1-2017/201463
- WO-A2-2007/111880
- WO-A2-2015/179002
- US-A1- 2016 067 196
- US-A1- 2017 242 976
- K JONKMAN ET AL: "Ketamine inhalation", BRITISH JOURNAL OF ANAESTHESIA., vol. 118, no. 2, 1 February 2017 (2017-02-01), GB, pages 268 - 269, XP055575004, ISSN: 0007-0912, DOI: 10.1093/bja/aew457

## Description

The present application relates to an inhaler for electronically supervised parenteral administration of a pharmaceutical composition, in particular to an inhaler that limits the abuse and misuse of the dry powder pharmaceutical composition with ketamine used in a treatment of a depression.

Depression, especially major depressive disorder, bipolar disorder and treatment-resistant depression (TRD) is a serious problem in a modern society. Many treatment options have been developed for treating depression, including monotherapy or combination therapy in a convenient for patients oral administration regimen. However, there is a relatively high percentage of patients that are treatment-refractory or partially or totally treatment-resistant to existing antidepressants. In practice, at present the only real choice in such severe cases can be electroshocks.

Ketamine is a known anesthetic and analgetic, used for anesthesia and in the treatment of chronic pain. Ketamine is a chiral compound and can exist as a racemate and as S-enantiomer (known as esketamine) or R-enantiomer (known as arketamine). Ketamine can form a pharmaceutically acceptable salts and in pharmaceutical applications is generally used as preferred hydrochloride salt. The optical rotation of an enantiomer varies between ketamine and its salts. For example, while esketamine free base is dextrarotatory S-(+), esketamine hydrochloride is levorotatory S-(-).

Since about one decade antidepressant activity of ketamine and its S-isomer (esketamine) is explored, especially in the treatment of treatment-resistant or treatment refractory depression (G. Serafini et al., The Role of Ketamine in Treatment-Resistant Depression: A Systematic Review., Current Neuropharmacology, 2014, 12, 444-461). Treatment-resistant depression is a term used in clinical psychiatry to describe cases of major depressive disorder that do not respond adequately to appropriate courses of at least two antidepressants in a suitable dose for a suitable time.

Data collected up to now show exceptional properties of ketamine and esketamine. The effect is very quick (after 2-3 hours from administration) and relatively long-lasting - a few days after single dose of a medicament. The rapidity of the clinical effect is surprisingly high and unexpected, since the effect of antidepressants present on the market appears after at least two weeks, even three to four weeks of day-to-day administration. Therefore, ketamine or esketamine could be used as a drug of first choice in patients with major depression with enhanced suicide risk that are resistant to existing oral antidepressants. The scale of the effect is also very high; about 2/3 of the patients with treatment-resistant depression is responsive to ketamine treatment.

The knowledge of the pharmacology of ketamine is still poor. As a dissociative anesthetic, the drug may exert dissociative and psychomimetic effects (DP). Available data show that this effects are correlated with systemic concentration of the drug. Dissociative and psychomimetic effects are among most often observed side-effects and significantly lower the comfort of patients. However, there are still groups of patients that respond to the treatment with ketamine without experiencing DP effects. Hence, still exists a therapeutic window, although narrow, for effective and safe use of ketamine in the treatment of depression without DP.

Ketamine undergoes extensive first-pass metabolism effect in the liver. Primarily, norketamine is produced as the initial metabolite. Norketamine is then metabolized to further metabolites. The knowledge about norketamine and further metabolites is still not full. On the level of action on NMDA receptor norketamine is many times less active than ketamine. Other metabolites are also mostly less active than ketamine.

Furthermore, little is known about toxicity of norketamine and other metabolites. This, in combination with high individual variations of their concentrations dependent on the status of hepatic enzymes, as a rule makes them undesired compounds. There are also reports on correlation of some hydroxylated metabolites of ketamine with psychotic and dissociative symptoms.

In previous studies ketamine and esketamine were administered in the treatment of depression intravenously or intranasally. Attempts of oral administration were generally unsuccessful or the effects were observed only after several weeks of administration.

Literature describes many examples of ketamine pharmacokinetics depending on the administration route.

Administration route with currently expected minimum level of metabolites is an intravenous one. After intravenous infusion of racemic ketamine at 0.5 mg/kg for 40 minutes, the parent drug maintains its systemic concentration about 200 ng/ml for about 40 minutes, afterwards the concentration falls down quickly with a half-period below 2 hours. Simultaneously, norketamine reaches its maximum concentration at the level of 10-20% of ketamine concentration. The percentage of area-under-curve (AUC) norketamine to ketamine is about 20-40%.

Oral administration is the administration route, after which maximum concentration of metabolites is expected. However, after oral administration the drug rapidly undergoes metabolism to norketamine. Norketamine level is equal to 500-1000% of ketamine level. Area-under-curve (AUC) for norketamine is even higher, exceeding 1000%.

The bioavailability of orally administered ketamine is very low (ca. 16-20%); while intravenous administration results in marked increase in ketamine bioavailability, it has also many disadvantages (e.g. long-time of infusion, patient discomfort, need for surveillance).

EP 3 111 978 A1 discloses an inhaler adapted to supply a formulation comprised in a container to a user, the inhaler being adapted to read information stored in data storage means of the container, and a system comprising such an inhaler and the container comprising the formulation. The container further comprising data storage means storing information, the container being adapted for supplying the formulation to a user, accordingly.

US2007/0287753A1 discloses the use of ketamine for treating treatment-resistant or refractory depression. The only formulation tested is the intravenous infusion, and transdermal administration is contemplated as well. Intranasal administration is only generally described, including intranasal administration of a dry powder aerosol formulation comprising finely divided powder of ketamine, a dispersant and bulking agent. However, with intranasal administration ketamine to oropharyngeal area significant amounts of ketamine will be swallowed by a patient by oral route and can undergo systemic metabolism to norketamine to cause undesired side effects.

DE102007009888 discloses the use of S-ketamine in the treatment of depression, in the dosage of 0.3 to 1.0 mg/kg. Although all possible administration routes are generally mentioned, the only formulation tested is intravenous infusion, mentioned as the preferred one.

WO2013/138322 discloses the use of esketamine in the treatment of treatment-refractory or treatment-resistant depression. Test for efficacy of esketamine was described in prophetic example with esketamine intravenous infusion.

WO2014/143646 and WO2014/152196 disclose pharmaceutical composition of esketamine in the form of the aqueous formulation of esketamine hydrochloride, preferably for nasal administration, for use in the treatment of treatment-refractory or treatment-resistant depression.

Mucoadhesive oral forms of esketamine and pharmacokinetics of esketamine after oral, intranasal and intravenous administration are described in WO2014/020155.

K. Jonkman et al., Anesthesiology 127 (4), 675-683, 10, 2017, studied on healthy volunteers the safety and feasibility of delivery of ketamine by inhalation of nebulized esketamine hydrochloride saline solution as a new route of ketamine administration. It has been found that inhaled ketamine bioavailability was reduced due to both dose-independent and dose-dependent impairment of pulmonary uptake. This was related to the high viscosity of esketamine; the viscosity of esketamine is three to four times greater than that of water. Because of this the administration via nebulization will be imprecise and non-reliable.

Singh et al., Biological Psychiatry 80:424-413, 2016, observed a rapid onset of robust antidepressant effects in patients with treatment resistant depression (TRD) after a 40-minute i.v. infusion of either 0.20 mg/kg or 0.40 mg/kg of esketamine. The lower dose may allow for better tolerability while maintaining efficacy.

The above illustrates the absolute medical need and importance of development of high-dose ketamine formulation that is both highly effective as well as convenient and easy to everyday self-administration by the patient including self-administration on out-patient basis to ensure high patient compliance. Such a formulation should first of all deliver therapeutic ketamine dose to the blood, should be characterized with high effectiveness, including rapid therapeutic effect and low risk of undesired effects, such as DP, due to precise dosing. Such a formulation should allow only a minimum level of systemic first-pass metabolites such as norketamine and hydroxylated metabolites, especially assure acceptable (es)ketamine to (es)norketamine ratio, both in view of avoiding reduction of ketamine level actually administered and unwanted metabolites effects.

The target was to achieve similar ketamine plasma concentration and hence similar antidepressant effect as that by Sing et al. with intravenous infusion of 0.20 mg/kg lasting 40 minutes using route of administration more convenient for a patient and producing less adverse effects.

The above problems have been solved by a high-dose and stable dry powder ketamine pharmaceutical composition for use in a method of treatment of depression by pulmonary administration route in a reliable, reproducible and convenient manner.

However in medicine there is often the need to control and/or monitor correct intake of medication, such as drugs and medicine which are typically prescribed for conditions concerning the nervous system, especially the brain, peripheral nerves, and spinal cord, due to a broad scope of medical situations. Within this scope there are drugs and medicine described and listed by FDA (United States Food and Drug Administration) as Neurology Drugs and Nervous System Drugs, including medication for pain relief purposes, such as opioids, and for which there is the need of rigorous control and monitoring of related effects. Concerning medicines for pain relief purposes, like opioids, those will be referred in this document their four subcategories: opiates, semi-synthetic opioids, synthetic opioids, and endogenous opioids.

Drugs and medicine described above may have a large spectrum of applications for different types of patients' conditions concerning, but not only, post-surgery, cancer treatments, as well as brain and nervous system conditions such as: Alzheimer's Disease, Attention Deficit Hyperactivity Disorder (ADHD) , Carpal Tunnel Syndrome, Huntington's Disease, dementia, memory loss, multiple sclerosis, muscular dystrophy, Parkinson's Disease, Tourette's Syndrome, and others, which they all require to careful follow related prescription and its effects.

FDA list of approved drugs for neurology and the nervous system includes a plurality of types of such medication supposed to be prescribed to patients by their medical doctors, as such medicines are considered "prescription drugs" or "prescription strong medicines", which include:
Opioids, as Opiate pain relievers, such as methadone, morphine, oxycodone (OxyContin) , fentanyl, sufentanil, levorphanol, oxymorphone, hydromorphone, meperidine (Demerol), and tramadol, as well as any chemical variation or combination of those;
Medicines that can be prescribed to be used with opiate pain relievers. Such medicines are usually prescribed to help pain medicine performance treating patient's symptoms, or they are specifically prescribed for certain types of pain. These medicines include, but not only: Bisphosphonates (e.g. dexamethasone, and prednisone) , Anti-inflammatory drugs and corticosteroids, local anesthetics (e.g. lidocaine, and capsaicin, to help pain in skin and surround tissues), Anticonvulsants, Antidepressants, and other medicines aiming to have similar effects. The medicines described above are given to patients in several ways depending of the specific conditions of each patient, and in general they are given by mouth. Although, in several circumstances, for example when the patient may have difficulties, or related problems, in swallowing capsules, these types of medicines may be taken in several other ways, including in cases when faster pain relief is needed.

In general, there are the following common ways of taking these medicines: by mouth: such as pills, capsules, tablets, liquids, and medicines that dissolve on the tongue or under the tongue, as well as through aerosol to be absorbed via the mouth and respiratory system into the body; using skin patches: the patch has medicine incorporated that is absorbed into the body through the skin; with rectal suppositories: such as in pills, or capsules, which are put inside the rectum and absorbed into the body; with needles: such as injections, or into a vein (IV - intravenous) . A patient taking medicines via IV may be able to use a Patient Controlled Analgesia (PCA) pump, which lets the patient control pain medicines in some limited ways.

Due to the specificity of these types of medicines there are potential problems and risks to the patients that are associated to hazardous situations of its misuse, including those of not following defined medical prescription, and which may occur by the following main failure modes:
1) Lack of self-control of the patient on the frequency and/or quantity of intake of the specific medicine or pharma product containing opioids to be administrated according medical prescription - classified as misuse of a pharmaceutical composition;
2) Deliberately wrong intake by the patient of the specific medicine or pharma product containing opioids, out of the quantity and/or frequency as medically prescribed - it is classified as an abuse of a pharmaceutical composition;
3) Unconscious intake, or deliberately conscious intake, by individuals which are not the intended patient and user of the specific medicine or pharma product according medical prescription.

The unsupervised administration of the pharmaceutical compositions or self-administration is therefore limited to pharmaceutical compositions that have a limited effect that even in a situation of abuse or breach of the administration scheme or protocol, their effects are to some extent predictable and risk of health damage limited. This problem was addressed in the prior art at many different ways in order to establish a controlled conditions for administration of a drug or pharmaceutical composition.

In the publication EP1973593, a drug storage and dispensing devices for dispensing a drug dosage form to a patient are disclosed. The dispensing device has a programmable lock-out feature for locking the dispensing device and is capable of detecting the identity of a user. Disclosed embodiment further provides a method for the treatment of subject, by administering to the subject a drug dosage form using a dispensing device .

In US2017/242976 a dispenser is disclosed comprising: a) a reclosable opening on, or for fitment on and/or around an opening of, a container having a cavity for receiving at least one unit of a product to be dispensed; b) a controller adapted for controlling the opening of the reclosable opening; c) a receiver adapted for receiving a user authentication signal; d) a power source for powering the controller and receiver; and wherein the dispenser only permits the opening of the reclosable opening upon the receiver receiving a user authentication signal. This publication discloses also a dispensing system, method of dispensing and a kit of parts including such a dispenser. Dispensers are particularly suited for dispensing pharmaceutical products to only the intended recipient and also to ensure compliance with dosage regimes.

US2010/100237 discloses a dispenser having means to dispense desired number of pills from a bulk supply of pills contained in the dispenser. The dispenser comprises of storage compartment having bulk supply of pills and having a discharge port emptying into counting compartment. The counting compartment contains first and second conveyors moving at first and second speed; wherein the second speed is greater than the first speed thereby enabling pill separation; the second conveyor discharges pills into dispensing compartment. Sensors are strategically placed along the conveyors to count pills discharged into dispensing compartment. A pill recovery system and apparatus is disposed inside the dispenser having means to recover pills remaining on conveyors upon completion of a dispensation cycle and deposit recovered pills back into the storage compartment for use in future dispensation cycles. A docking station having receptacles to accommodate dispenser is provided. Docking station has communication ports enabling two-way communication with personal computer. The dispenser has multiple security features including locking mechanisms at inlet and outlet; and internal circuitry that is responsive to the 'disable' electronic signal originating from dispenser's internal clock and remote server in communication link with the dispenser.

US2013/226339 discloses systems and methods for detecting a likely misuse of a medicament by a user. The system includes a computer communicatively coupled with a dispensing device. The computer receives a usage pattern of a medicament by the user as indicated by the dispensing device and a result of a test correlating with an actual consumption of the medicament by the user. Based on the usage pattern, the computer computes an estimated result of a test corresponding to the at least one predetermined test. Based on a comparison between the estimated result and the test result, a determination is made as to whether the user has likely misused the medicament.

US2014/297028 discloses a battery-powered, rechargeable, handheld device dispenses medication film strips in a controlled way. The device is password protected, restricts doses, communicates wireless with a server host so that a doctor and pharmacist can monitor the device and can destroy the medication remotely if the device is lost, stolen or tampered with. The device may be trackable by GPS location. Software can track the device as well as a doctor's caseload to assure compliance with regulations. The device is an automated device that uses sophisticated electronics to remove the human element and force the patient to adhere to a programmed regimen. The device also simplifies the process of monitoring and tracking for the doctor. The problem of accidental child exposure is eliminated. The problem of abuse and diversion of the drug is effectively controlled and limited. Nothing is left to human interpretation or variability in practice.

WO2019/038580 discloses a medicament dispenser for delivering a medicament to a user, the medical dispenser comprising one or more internal storages for storing one or more medicaments; a dispensing unit configured to access said one or more internal storages and dispense the medicament based on a predefined dispensing protocol; a control unit comprising a user recognition unit, adapted to collect user authentication data; and a communication module, configured to send/receive said user authentication data and a delivery control data associated to said predefined dispensing protocol to/from a remote server; wherein said control unit is configured to enable/disable said dispensing unit based on said user authentication data and said delivery control data. Also disclosed is a medicament re-filling apparatus for the use with a medicament dispenser upon authentication and validation of its user condition.

As indicated above the administration of the pharmaceutical compositions without a direct control of the authorised entity responsible for controlling the administration process is the part of therapy that relies solely on the patient's sense of responsibility for his/her wellbeing. Unsupervised administration of the pharmaceutical compositions is also a main factor leading to a misuse or abuse of pharmaceutical compositions. It is also a major reason why the applied therapies are not effective, as the pharmaceutical compositions are often administered outside the planned scheme by patients not following the assigned protocol.

The invention is as defined in claims 1 to 15.

The invention is disclosed in the context of the system that provides a secure way of controlling the administration scheme, removing problems existing in the prior art. Using authorisation tokens removes a necessity of time controlling, computing engaging means of personal authentications. It eliminates the need for storing personal biometric data, remembering passwords and complicated authorisation procedures.

The invention provides an inhaler with dry powder pharmaceutical composition comprising ketamine or a pharmaceutically acceptable salt thereof as a medicine for use in a method of treatment of depression by pulmonary administration.

In another aspect, the invention provides inhaler with ketamine or its pharmaceutically acceptable salt for use in a method of treatment of depression, wherein ketamine or its pharmaceutically acceptable salt is administered by pulmonary route as a dry powder pharmaceutical formulation.

The inhaler according to the present invention provides a secure way of controlling the administration scheme, removing problems existing in the prior art. Using authorisation tokens removes a necessity of time controlling, computing engaging means of personal authentications. It eliminates the need for storing personal biometric data, remembering passwords and complicated authorisation procedures.

Providing a secure authorisation with a use of a personal device takes advantage of the new behavioural pattern observed when the mobile phone is a device always present with a person.

Further advantage of the system with an inhaler according to the present invention is providing a system that provides a high quality monitoring data for a physician that refers to the administration process eliminating assumptions found in the prior art that dispensing a drug equals to a proper administration of a drug.

Still further advantages, as well as features and ways of carrying out the present invention will become apparent from the following detailed description of a preferred embodiment, presented by way of a non-limiting example, making reference to the figures of the accompanying drawings, in which:
- Fig. 1: presents NGI deposition data for the composition of Example 1;
- Fig. 2: presents NGI deposition data for the composition of Example 2;
- Fig. 3: presents NGI deposition data for the composition of Example 3;
- Fig. 4: presents NGI deposition data for the composition of Example 4;
- Fig. 5: presents NGI deposition data for the composition of Example 5;
- Fig. 6: presents NGI deposition data for the composition of Example 6;
- Fig. 7: shows esketamine plasma concentration vs time after administration of various single doses of dry powder composition of Example 2;
- Fig. 8: shows esketamine plasma concentration vs time after administration of a sequence of single doses of dry powder composition of Example 2; and
- Fig. 9: presents adverse effect distribution after administration of dry powder composition of Example 2 (PART A).

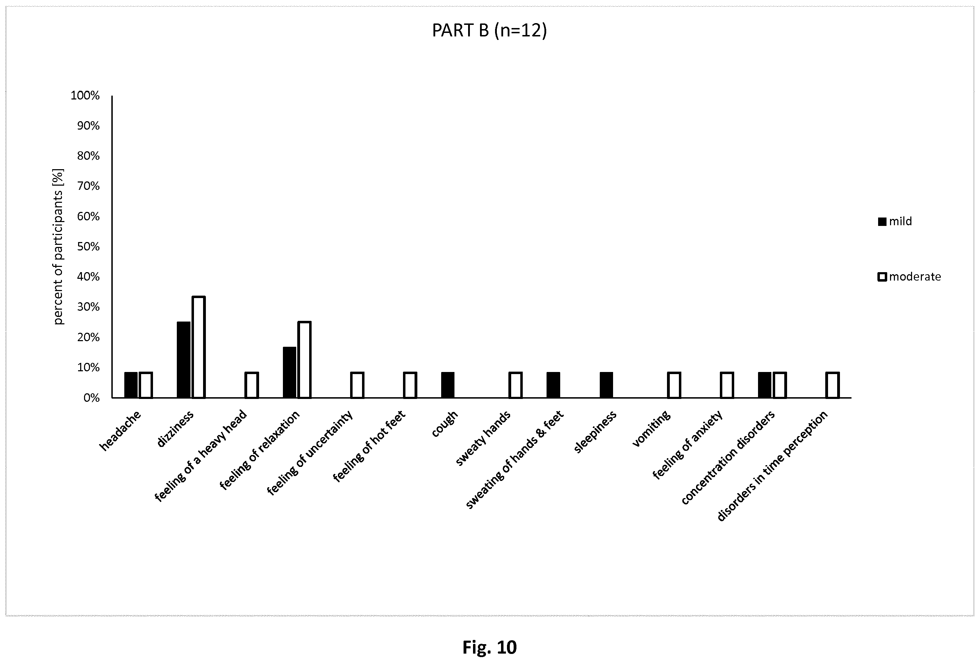

- Fig. 10: presents adverse effect distribution after administration of dry powder composition of Example 2. (PART B).
- Fig. 11: is a schematic diagram of the first embodiment of the system for electronically supervised parenteral administration of a pharmaceutical composition with an inhaler according to the invention;
- Fig. 12: is a schematic diagram of the control signal generated by the control station to control an inhaler according to the invention;
- Fig. 13: is a schematic diagram of the inhaler according to the invention;
- Fig. 14: is a schematic diagram of the inhaler with a measurement unit according to the invention;
- Fig. 15: is a schematic diagram of the ranking matrix for use in the system for electronically supervised parenteral administration of a pharmaceutical composition with an inhaler according to the invention;
- Fig. 16: is a schematic diagram of the process of assigning a value of quality measure to a measured physical property using a ranking matrix in the system for electronically supervised parenteral administration of a pharmaceutical composition with an inhaler according to the invention;
- Fig. 17.: is a schematic diagram of the second embodiment of the system for electronically supervised parenteral administration of a pharmaceutical composition with an inhaler according to the invention;
- Fig. 18: is a schematic diagram of the another embodiment of the inhaler according to the invention;

An embodiment of the invention is an inhaler with a dry powder pharmaceutical composition comprising ketamine or its pharmaceutically acceptable salt as a medicine for use in a method of treatment of depression by pulmonary administration, i.e. administration via pulmonary route.

The inhaler may have ketamine or its pharmaceutically acceptable salt for use in a method of treatment of depression, wherein ketamine or its pharmaceutically acceptable salt is administered by pulmonary route as a dry powder pharmaceutical formulation.

Preferably, esketamine, especially esketamine hydrochloride, is self-administered pulmonary by a patient by inhalation of a dry powder esketamine composition or formulation in a sequence of administrations consisting of multiple single doses (inhalation events), such as at least 3 single doses, each inhalation event consisting of multiple puffs, such as 1, 2, 3 or 4 puffs, preferably in 3 or 4 puffs, said sequences being separated from each other by a break period without any inhalation (rest period). Preferably, such as sequence lasts at least 30 minutes, for example lasts 30 minutes, and includes 3 sequences of administration and break periods between are preferably equal, i.e. are 15 minutes break (rest) period.

Preferably, esketamine, especially esketamine hydrochloride, is self-administered pulmonary by a patient by inhalation of a dry powder esketamine composition or formulation in a sequence lasting 30 minutes consisting of 3 single doses (inhalation events), each inhalation event consisting of 3 or 4 puffs, wherein each puff corresponds to esketamine nominal dose of 4 mg in the dry powder composition or formulation. Such a composition or formulation is described in Example 2 below. Between such each inhalation event (single dose) there is provided a break period without any inhalation, preferably there are two equal breaks lasting about 15 minutes, i.e. first single dose is administered at time 0, second single dose is administered after about 15 minutes and the third single dose is administered at 30 minute. Such a sequence allows to obtain plasma concentration profile that provides plasma concentration infusion at the level having antidepressant effect, as known from prior art tests of intravenous infusions.

The term "ketamine" encompasses racemic ketamine and its enantiomers esketamine and arketamine, both as a free base and pharmaceutically acceptable salts thereof.

In a preferred embodiment ketamine is esketamine.

In another embodiment, ketamine is racemic ketamine.

Preferred pharmaceutically acceptable ketamine salt is hydrochloride.

In a most preferred embodiment, the composition comprises esketamine hydrochloride.

In another embodiment, the composition comprises racemic ketamine hydrochloride.

Preferably, ketamine, especially esketamine such as esketamine hydrochloride, is self-administered pulmonary by a patient by inhalation of a dry powder ketamine composition or formulation in a sequence of administrations consisting of multiple single doses (inhalation events), such as at least 3 single doses, each single dose or inhalation event consisting of multiple puffs, such as 1, 2, 3 or 4 puffs, preferably in 3 or 4 puffs, said sequences being separated from each other by a break period without any inhalation (rest period). Preferably, such as sequence lasts at least 30 minutes, for example lasts 30 minutes, and includes 3 sequences of administration and break periods between are preferably equal, i.e. are 15 minutes break (rest) period.

Preferably, esketamine such as esketamine hydrochloride, is self-administered pulmonary by a patient by inhalation of a dry powder esketamine composition or formulation in a sequence lasting 30 minutes consisting of 3 single doses (inhalation events), each inhalation event consisting of 3 or 4 puffs, wherein each puff corresponds to esketamine nominal dose of 4 mg in the dry powder composition or formulation. Such a composition or formulation is described in Example 2 below. Between such each inhalation event (single dose) there is provided a break period without any inhalation, preferably there are two equal breaks lasting about 15 minutes, i.e. first single dose is administered at time 0, second single dose is administered after about 15 minutes and the third single dose is administered at 30 minute. Such a sequence allows to obtain plasma concentration profile that provides plasma concentration infusion at the level having antidepressant effect, as known from prior art tests of intravenous infusions.

The term "medicine" as used herein can be used interchangeably with the term "medicinal product". It should be understood that "medicine" and "medicinal product" have essentially the same meaning in terms of the invention.

The term "treatment-resistant or treatment refractory depression" (TRD) is well known in the art and means depression in patients not responding to at least two prior attempts of adequate antidepressive treatment using commonly known antidepressant therapies. The term is generally described for example in US8,785,500 and US2015/0056308.

The term "bipolar disorder" is well known in the art and means a disorder that causes periods of depression and periods of abnormally elevated mood.

The term "major depression" is well known in the art and means a disorder characterized by at least two weeks of low mood that is present across most situations.

In one aspect the composition comprises from 2 mg to 100 mg of ketamine calculated as a free base per nominal unit dose.

In a particular embodiment, the composition comprises from 2 mg to 60 mg of ketamine, especially 2 mg to 40 mg of ketamine, such as from 3 mg to 15 mg of ketamine, calculated as a free base, per nominal unit dose.

In another embodiment, the composition comprises further one or more additives selected from the group consisting of a carbohydrate bulking agent in the amount of 30 to 95% by weight and a stabilizing agent in the amount of 0.2 - 3% by weight, with respect to the total weight of the composition.

The composition comprises ketamine, especially esketamine hydrochloride, having median particle diameter d50 of 1 - 10 µm, such as 1 - 8 µm, especially 3 µm, d10 of 0.2 - 5 µm and d90 of 3 - 35 µm.

Median particle size d50 is a parameter obtained by laser diffraction technique with dry dispersion using Sympatec HELOS laser diffractometer attached with ASPIROS feeder. For measurement, raw ketamine, especially esketamine hydrochloride, is dispersed with pressure 3.0 bar in total amount of 30 mg per sample.

The composition is a dry powder formulation for administration using dry powder inhalers. Conventional and typical dry powder inhalers can be used for this purpose.

The term "dry powder" is known for a skilled person and should be understood in a manner conventional in the art as a solid mix of particles that is fluidized when the patient inhales after actuation of the inhaler device.

The term "nominal unit dose" relates to the ketamine dose as present (loaded) in the composition that is destined for a single administration. The nominal unit dose can be a measured dose of the dry powder to be ready for the patient to take, contained in a single unit, such as a capsule or single compartment in a blister, or a dose to be taken from for delivery from the multi-dose dry powder reservoir.

The term "emitted dose" relates to the proportion of the nominal unit dose that exits/leaves the device after inhalation by a patient.

The dry powder pharmaceutical composition or formulation may comprise further pharmaceutical excipients., i.e. one or more additives selected from the group consisting of a carbohydrate bulking agent (a carrier) in the amount of 30 to 95% by weight and a stabilizing agent in the amount of 0.2 - 3% by weight, with respect to the total weight of the composition.

Suitable carbohydrate bulking agent (a carrier) can be lactose, D-mannitol, glucose monohydrate, trehalose, especially trehalose dihydrate, erythritol, dextrose, maltose, sorbitol or xylitol. Especially convenient bulking agent is milled lactose, such as lactose monohydrate or anhydrous lactose, especially lactose monohydrate, having suitable granulometry. Suitable granulometry is defined as having d50 30 - 200 µm (Sympatec HELOS) as the main coarse fraction (especially 80 µm). Examples of suitable lactose monohydrate commercial grades are Lactohale 200 (LH200), Lactohale 100 (LH100) and Lactohale 200LP. Various types of inhalers may require appropriate selection of lactose grade most suitable for performance thereof. Such a selection is within common skills of a skilled person.

Typical amount of the bulking agent in the composition is 30 - 95% by weight, especially 30 to 80% by weight, with respect to the total weight of the composition.

Pharmaceutical excipients/additives include also a stabilizer (also called force control agent - FCA), i.e. a substance that reduces adhesion and cohesion. Suitable stabilizers are for example magnesium stearate, lecithin, and aminoacids, such as leucine. Especially preferred stabilizer is magnesium stearate.

Stabilizer "disturbs" the weak binding forces between the small particles and thus helps to keep the particles separated, reduces self-adhesion of small particles and also adherence to other particles in the formulation if such other particles are present, reduces the adhesion to the inner surfaces of the inhaler, as well as improves rheological properties of powder - powder flowability.

The amount of the stabilizer in the composition is 0.2 - 3% by weight, especially 0.8% by weight, with respect to the total weight of the composition.

Composition or formulation is prepared by blending in a high-shear mixer a bulking agent/carrier of suitable granulometry with a stabilizer, and then adding ketamine, especially esketamine hydrochloride, of suitable granulometry and again blending in a high-shear mixer.

Alternatively, ketamine, especially esketamine hydrochloride, of suitable granulometry is co-processed (blended) with a stabilizer in a high-shear mixer, and then the bulking agent/carrier is added and again mixed in a high-shear mixer.

The composition is a dry powder formulation for administration using dry powder inhalers. Conventional and typical dry powder inhalers can be used for this purpose.

The formulation may be administered by three device categories: single-unit dose inhaler in which each dose, such as in a capsule, is loaded into the device before use; a multi-dose reservoir inhaler in which a bulk supply of dry powder with plurality of doses is preloaded into the device; and a multi-unit dose inhaler in which a plurality of single doses are individually sealed in separate compartments such as in a blister cavity, and discharged each time the device is actuated. Preferred is the multi-unit dose inhaler in which a plurality of single doses are individually sealed, such as in the blister, and discharged each time the device is actuated.

In one embodiment as defined above, the medicine for administration via pulmonary route is a blister with plurality of individual nominal unit doses premetered and individually sealed. One preferred example of such an inhaler is Diskus type inhaler.

In another embodiment as defined above, the medicine for administration via pulmonary route is a capsule with a single nominal unit dose.

In another embodiment as defined above, the medicine for administration of a single dose via pulmonary route is a multi-dose powder reservoir.

The composition provides emitted dose of at least 1.0 mg of ketamine calculated as a free base, corresponding to 1.2 mg of ketamine hydrochloride.

The composition provides the fraction of the dose delivered locally directly to the lungs that is at least 40%, such as from 40 to 50%, especially 40% to 60%, especially up to 85%, of the emitted unit dose.

Emitted dose is the portion of nominal unit dose that is emitted from the inhaler device and leaves the inhaler device as an aerosol and hence is available to the patient.

Only part of emitted dose reaches the lungs and thus circulating blood of a patient as the dose delivered to the lungs (also called Fine Particle Dose - FPD) or fraction delivered to the lungs (also called Fine Particle Fraction - FPF). Some part reaches gastrointestinal tract via oropharyngeal and oral routes, i.e. is swallowed, and is accessible for undesired first-part metabolism.

It has been surprisingly found that in spite of well-known problems with inhalation dry powder formulation of high doses of an active substance for pulmonary administration, the uniform and stable high-dose ketamine, especially esketamine hydrochloride dry powder composition can be obtained that when administered by pulmonary route provides therapeutic ketamine level in the circulating blood of a patient, i.e. at least 50 to 100 ng/ml, such as 70 to 100 ng/ml, such as 70-80 ng/ml, such as about 100 ng/ml. Therapeutic ketamine level relates to the level in the blood that is effective in the treatment of depression, especially major depressive disorder, such as treatment resistant or treatment-refractory depression, and may be dependent on the subject, gender, age, severity of the disease, the type of the inhaler, and may vary depending on whether ketamine is racemic ketamine or enantiomeric ketamine.

The fraction of the emitted dose delivered to the lungs is surprisingly high, in contrast with typical inhalation compositions wherein the standard is that only 15 to 20% of the emitted dose is delivered to the lungs.

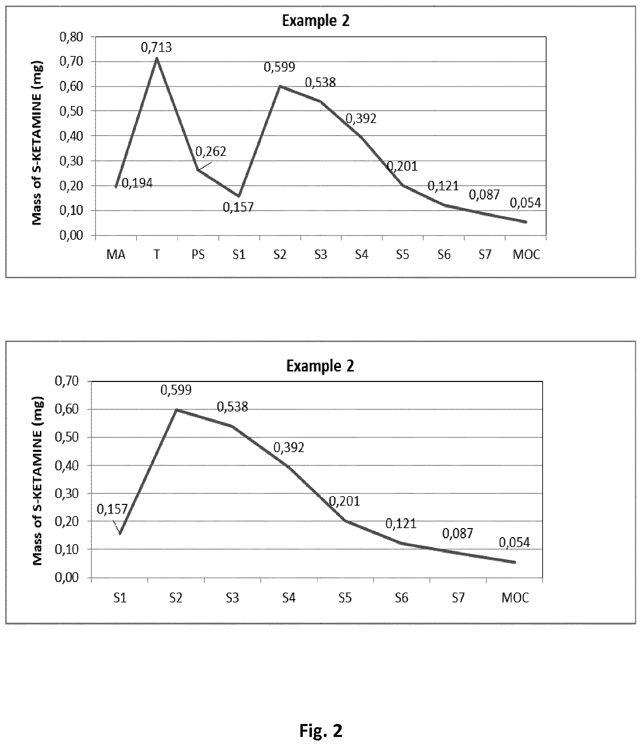

The fraction of the emitted dose delivered locally directly to the lungs (also called Fine Particle Fraction - FPF) can be determined using well-known and conventional methods and assays. Such methods and assays include any of those described in European Pharmacopeia 9.0, Chapter 2.9.18, Preparations for inhalation; Aerodynamic assessment of fine particles for determination of Fine Particle Dose. In particular, the Next Generation Pharmaceutical Impactor (NGI) (Ph. Eur. Apparatus E) can be used to assess and control the aerodynamic particle size distribution (APSD). The NGI apparatus is as presented in Figs 2.9.18.-12 and 2.9.18.-13 on page 333 of European Pharmacopeia 9.0.

Emitted dose and fine particle dose and fraction (FPF and FPD) are strongly dependent on two factors i.e. on the formulation and on the device. For the device the most discriminatory factor for emitted dose is resistance. The resistance of a dry powder inhaler (DPI) is an intrinsic value which depends on the design of the inhalation channel, the metering cup and the air inlets. DPIs can be classified into four resistance groups (low, medium, medium-high, high) with respect to the inhalation flow required to produce a pressure drop of 4 kPa. This value was chosen because it is the one recommended by pharmacopoeia for the in vitro characterization of the dose emitted from a DPI. Additionally for capsule-based DPIs can be limited by the powder retention in the capsule and device, which lead to reduction in the emitted dose.

Emitted dose testing is relatively straightforward. The device is 'fired' into a sampling apparatus that enables the capture of the measured dose on a filter. The aerodynamic particle size distribution of inhaled products is measured using the technique of multistage cascade impaction, here Next Generation Impactor (NGI). The collected quantity of active ingredient is determined further by HPLC analysis. The inhalers are tested at a predetermined flow rate, and the pressure drop across the inhaler is 4.0 kPa in line with the Ph Eur.

Efficient particle capture is ensured by coating the particle collection surface of each of stages 1-7, as well as the MOC and the pre-separator base, with a coating substance. The central cup of the pre-separator is filled with adequate diluent.

After discharging the powder to the NGI (Number of actuations per impactor n=1 for one analysis) by opening the two-way solenoid valve for the required time at flow control which generate pressure drop across the inhaler 4 kPa the following operations are performed:
I. Stages 1 to 7 and MOC. Each stage is washed with appropriate diluent (extraction of drug substance). NGI tray loaded with the cups on a Copley Gentle Rocker is gently shaken for 10 minutes.
II. Mouthpiece adapter. Deposited inhalation powder on adapter is rinsed with appropriate diluent a volumetric flask and sonicated for 10 minutes.
III. Induction port. Deposited inhalation powder from induction port is rinsed with appropriate diluent into a volumetric flask and sonicated for 10 minutes.
IV. Preseparator. Deposited inhalation powder from these component is rinsed with appropriate diluent into a volumetric flask and sonicated for 10 minutes.

Finally collected samples from each stage of impactor are filtered analyzed by high-performance liquid chromatography

Composition has an appropriate ketamine, in particular esketamine hydrochloride pharmacokinetics profile that enables achievement of approximately 50 to 100 ng/ml of the ketamine plasma concentration over 40 minutes after pulmonary administration directly to the lungs by inhalation. Said plasma concentration corresponds to antidepressive effect. Maintaining this concentration over time mimics 40-minute intravenous infusion known to be effective in depression and well-tolerated.

### Examples

### General manufacturing procedure:

A sum of lactose monohydrate and magnesium stearate are sieved through 0.25 mm mesh and mixed in high-shear mixer for 3 minutes. Obtained mixture is sieved with active substance through 0.5 mm mesh and mixed in high-shear mixer for 5 minutes.

To eliminate electrostatic charges, antistatic PE bags are used during the process.

### Vacuum filling process (blisters):

Vacuum-drum technology dose forming process is used for blister filling. The blister cavity is in volume range of 15 to 45 mm3 (especially ca. 30 mm3). Powder which is filled into cavity is in amount of 10 - 30 mg (especially 23 mg).

During process parameters of vacuum-drum device are:
Vacuum pressure: -0 - 500 mBar, especially 50 - 400 mBar
Fluidization pressure: - 0.1 - -0.4 Bar
Fluidization time: 50 - 2000 ms, especially 50 -300 ms
Filling time: 50 - 700 ms, especially 50 - 400 ms
Sealing time: 100 - 600ms

Sealing tests of filled blisters are performed under vacuum.

Finally, the blister strips are coiled into the inhaler.

### Filling process (capsules):

Capsules to be filled are placed in the sockets closed ends down. Powder is discharged from the dosator and comes directly to the capsules. The powder with which the capsules are to be filled is placed in the dosator, may be tamped and discharged into the capsules.

During the process parameters of capsule filling device are:
Rotation: 1- 70 rpm
Tamping high: 1 - 10mm
Dosator high: 1 - 250mm

Finally, the filled capsules are mounted into the inhaler.

### Ketamine dry inhalation powder for blisters and capsules

The following compositions has been prepared in accordance with the above general procedure in the scale of 0.9 kg.

### Example 1

| **Component** | **Amount (mg/unit)** |
|---|---|
| Esketamine hydrochloride | 3.45 (corresponds to 2.99 mg esketamine) |
| Lactose monohydrate LH200 LP | 19.16 |
| Magnesium stearate | 0.39 |

### Example 2

| **Component** | **Amount (mg/unit)** |
|---|---|
| Esketamine hydrochloride | 4.61 (corresponds to 4 mg esketamine) |
| Lactose monohydrate LH200 LP | 18.20 |
| Magnesium stearate | 0.18 |

### Example 3

| **Component** | **Amount (mg/unit)** |
|---|---|
| Esketamine hydrochloride | 5.06 (corresponds to 4.39 mg esketamine) |
| Lactose monohydrate LH200 LP | 17.581 |
| Magnesium stearate | 0.359 |

The compositions have been found uniform in accordance with requirements of Ph.Eur.2.9.40. Average esketamine hydrochloride content (n=10) was in the range 92.5% - 107.5% of nominal dose.

The process has been found scalable to the scale of 1.8 kg.

**Aerodynamic Particle Size Distribution (APSD)** test of the compositions of the Examples 1, 2 and 3 of the composition.

The compositions of Examples 1, 2 and 3 have been tested using the Next Generation Pharmaceutical Impactor (NGI) (Ph. Eur. Apparatus E) in accordance with the procedure for powder inhalers.

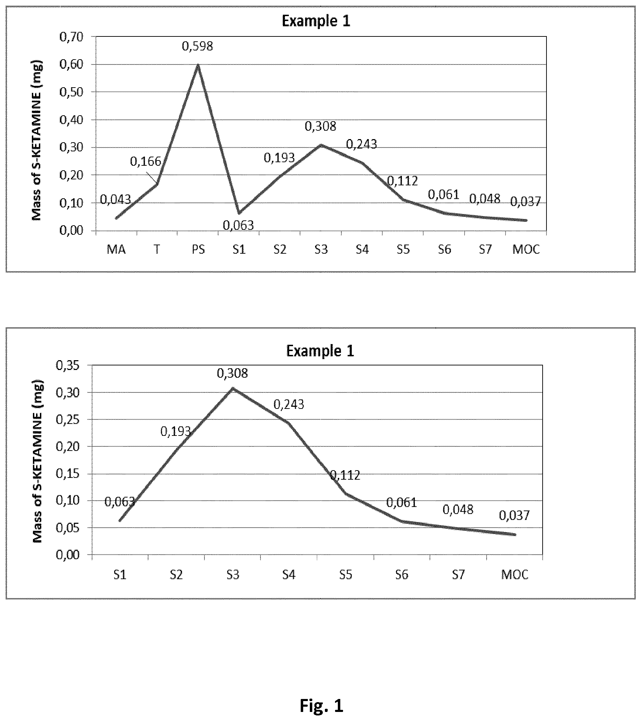

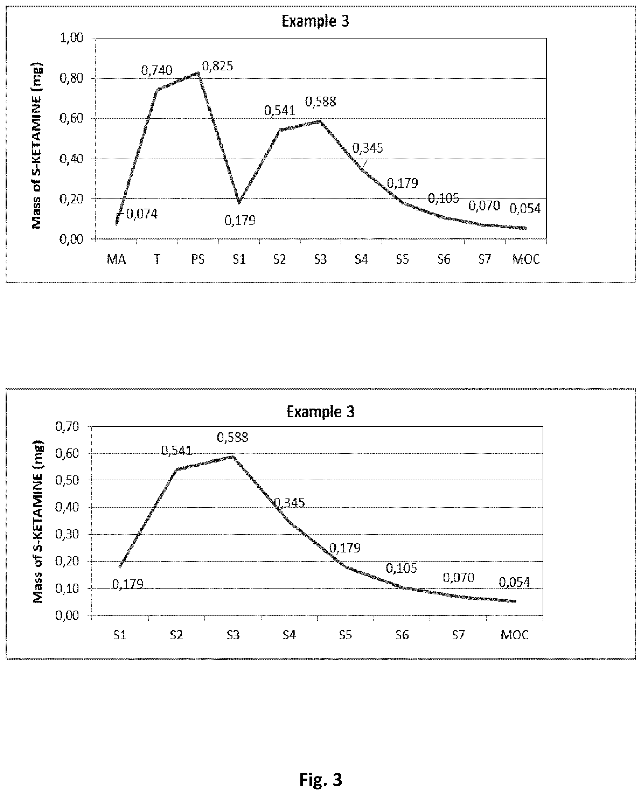

The results of the tests are presented in Table 1 below and in Fig. 1 (Example 1), Fig. 2 (Example 2) and Fig. 3 (Example 3) of the drawing, wherein upper diagrams present APSD data for the whole NGI and bottom diagrams present APSD data for stages 1-7 and MOC. The following abbreviations are used for the results of the tests:
MA - mouth adapter
T- induction port
PS - Pre-separator
S1-S7 - stages of NGI
MOC - micro-orifice collector
ISM - Impactor sized mass; mass entering the impactor excluding non-sizing portions
MMAD (µm) - mass median aerodynamic diameter. Defined as the diameter at which 50% of the particles by mass are larger and 50% are smaller.
GSD - geometric standard deviation. Measure of the spread of an aerodynamic particle size distribution
FPF - fine particle fraction (%)
FPD - fine particle dose

**Table 1. NGI deposition data**

| Example No | 1 | 2 | 3 |
|---|---|---|---|
| MA [mg] | 0.043 | 0.194 | 0.074 |
| T | 0.166 | 0.713 | 0.740 |
| PS | 0.598 | 0.262 | 0.825 |
| S1 | 0.063 | 0.157 | 0.179 |
| S2 | 0.193 | 0.599 | 0.541 |
| S3 | 0.308 | 0.538 | 0.588 |
| S4 | 0.243 | 0.392 | 0.345 |
| S5 | 0.112 | 0.201 | 0.179 |
| S6 | 0.061 | 0.121 | 0.105 |
| S7 | 0.048 | 0.087 | 0.070 |
| MOC | 0.037 | 0.054 | 0.054 |
| ISM (mg) | 1.00 | 1.99 | 1.88 |
| Total Mass on Impactor (mg) | 1.07 | 2.15 | 2.06 |
| Total Mass on System (mg) | 1.87 | 3.32 | 3.70 |
| Mass on Impactor/Actuation (mg) | 1.07 | 2.15 | 2.06 |
| Mass on System/Actuation (mg) | 1.87 | 3.32 | 3.70 |
| FPD ≤5.0 mcm (mg) esketamine | 1.0 | 1.7 | 1.6 |
| FPF ≤5.0 mcm (%) | 49.0 | 51.0 | 44.0 |
| MMAD (mcm) | 2.6 | 2.9 | 3.0 |
| GSD | 1.8 | 1.8 | 1.8 |

The obtained results showed a product with expected quality attributes.

The composition demonstrated appropriate homogeneity and a very high level of fine particle fractions, with:
FPF > 49%, FPD 1.0 mg; and emitted dose: 2.3 mg, for Example 1
FPF > 47%, FPD: 1.7 mg; and emitted dose: 3.6 mg, for Example 2, and
FPF > 44%, FPD: 1.6 mg; and emitted dose: 3.9 mg, for Example 3.

### Esketamine dry inhalation powder for capsules

The following compositions has been prepared in accordance with the above general procedure in the scale of 0.9 kg.

### Example 4

| **Component** | **Amount (mg/unit)** |
|---|---|
| Esketamine hydrochloride | 5.00 (corresponds to 4.34 mg esketamine) |
| Lactose monohydrate LH200 LP | 19.8 |
| Magnesium stearate | 0.2 |

### Example 5

| **Component** | **Amount (mg/unit)** |
|---|---|
| Esketamine hydrochloride | 10.00 (corresponds to 8.67 mg esketamine) |
| Lactose monohydrate LH200 LP | 39.6 |
| Magnesium stearate | 0.4 |

### Example 6

| **Component** | **Amount (mg/unit)** |
|---|---|
| Esketamine hydrochloride | 20.00 (corresponds to 17.34mg esketamine) |
| Lactose monohydrate LH200 LP | 79.2 |
| Magnesium stearate | 0.8 |

**Aerodynamic Particle Size Distribution (APSD)** test of the compositions of Examples 4, 5 and 6.

The compositions of Examples 4, 5 and 6 have been tested using the Next Generation Pharmaceutical Impactor (NGI) (Ph. Eur. Apparatus E) in accordance with the procedure for powder inhalers.

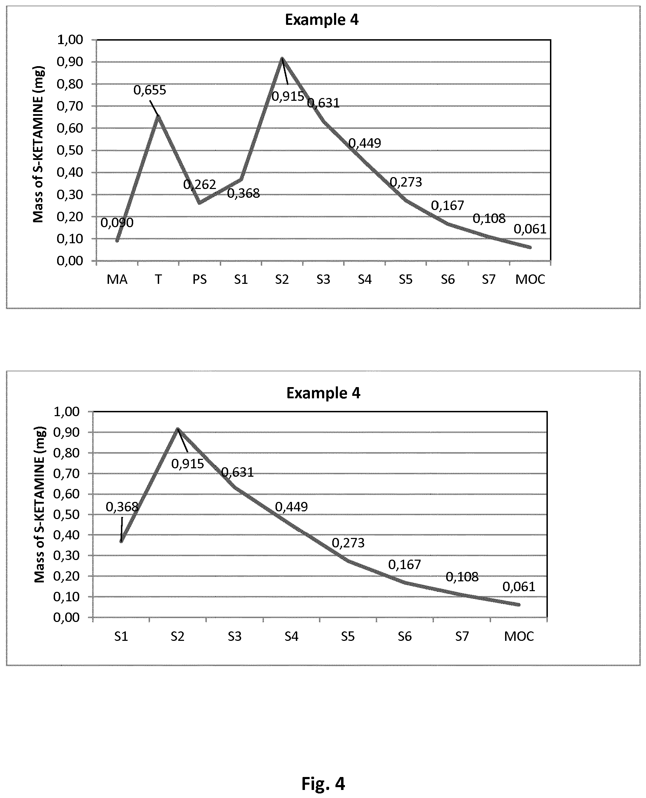

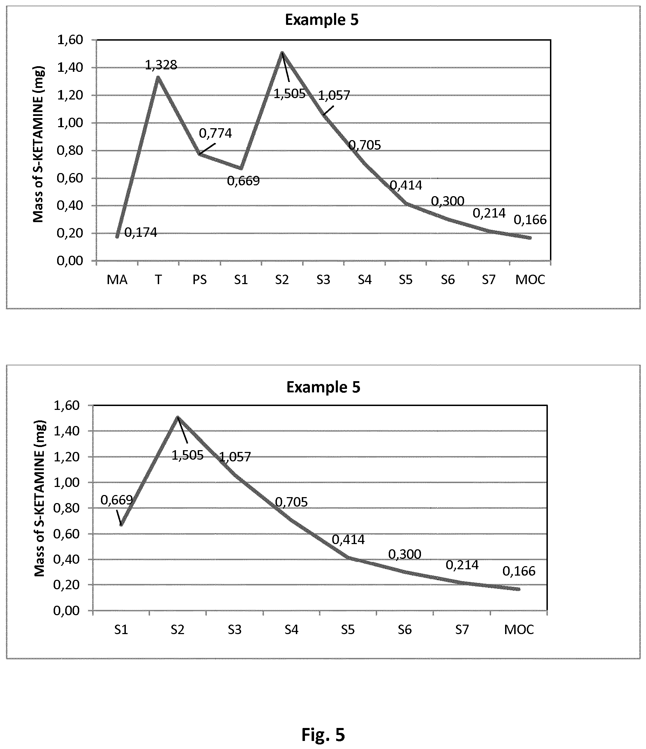

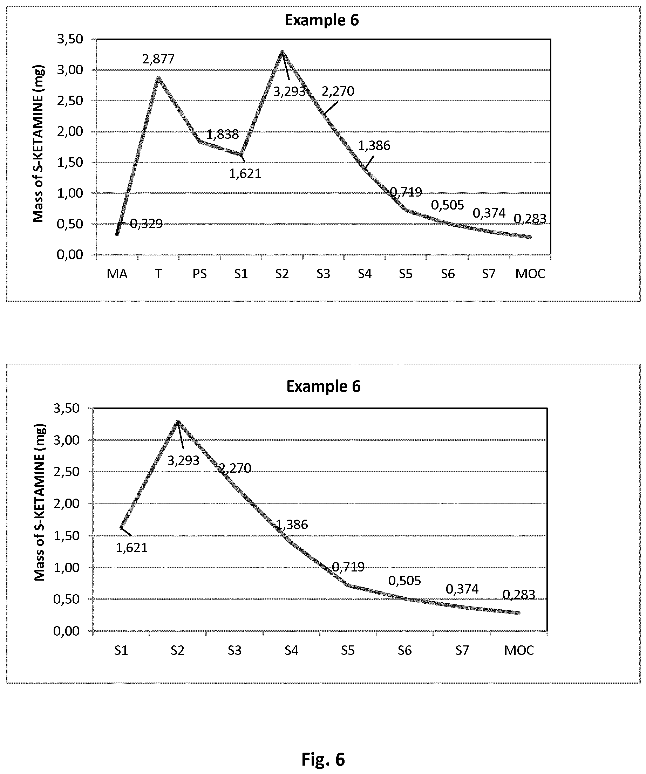

The results of the tests are presented in Table 2 below and in Fig. 4 (Example 4), Fig. 5 (Example 5) and Fig. 6 (Example 6) of the drawing, wherein higher diagrams present APSD data for the whole NGI and lower diagrams present APSD data stages 1-7 and MOC.

**Table 2. NGI deposition data**

| Example No | 4 | 5 | 6 |
|---|---|---|---|
| MA [mg] | 0.090 | 0.174 | 0.329 |
| T | 0.655 | 1.328 | 2.877 |
| PS | 0.262 | 0.774 | 1.838 |
| S1 | 0.368 | 0.669 | 1.621 |
| S2 | 0.915 | 1.505 | 3.293 |
| S3 | 0.631 | 1.057 | 2.270 |
| S4 | 0.449 | 0.705 | 1.386 |
| S5 | 0.273 | 0.414 | 0.719 |
| S6 | 0.167 | 0.300 | 0.505 |
| S7 | 0.108 | 0.214 | 0.374 |
| MOC | 0.061 | 0.166 | 0.283 |
| ISM (mg) | 2.61 | 4.36 | 8.83 |
| Total Mass on Impactor (mg) | 2.97 | 5.03 | 10.45 |
| Total Mass on System (mg) | 3.98 | 7.30 | 15.49 |
| Mass on Impactor/Actuation (mg) | 2.97 | 5.03 | 10.45 |
| Mass on System/Actuation (mg) | 3.98 | 7.30 | 15.49 |
| FPD ≤5.0 mcm (mg) esketamine | 2.4 | 3.9 | 7.9 |
| FPF ≤5.0 mcm (%) | 59 | 54 | 51 |
| MMAD (mcm) | 3.0 | 3.0 | 3.2 |
| GSD | 1.9 | 1.9 | 2.6 |

The obtained results showed a product with expected quality attributes.

The invented formulation demonstrated appropriate homogeneity and a very high level of fine particle fractions, with:
FPF > 59%, FPD 2.4 mg; emitted dose: 4.2 mg, for Example 4
FPF > 54%, FPD: 3.9 mg; emitted dose: 7.1 mg, for Example 5, and
FPF > 51%, FPD: 7.9 mg; emitted dose: 16.5 mg, for Example 6.

The dry powder pharmaceutical composition provided emitted esketamine hydrochloride dose at the level up to 97%, such as up to 85% of the nominal dose and at least 40% of fine particle fraction (fraction delivered to the lungs) for emitted esketamine dose.

### Example 7

### Pharmacokinetics of inhaled esketamine dry powder in healthy volunteers

Esketamine hydrochloride dry powder formulation of Example 2 was administered to healthy volunteers pulmonary, i.e. directly to the lungs using dry powder inhaler (DPI) (by self-administration).

One puff of dry powder formulation contained 4.6 mg of esketamine hydrochloride, corresponding to 4 mg of esketamine free base and excipients 18.22 mg of lactose monohydrate and 0.18 mg of magnesium stearate.

A single dose was an inhalation events consisting of 1 to 6 puffs, i.e. 4 to 24 mg of esketamine free base nominal dose.

In part A of the study, designed as a one-centre single ascending dose, the medicine was delivered in a single dose once daily (up to 6 consecutive puffs) to 18 healthy volunteer subjects. Subjects were divided into 6 cohorts, cohorts receiving 1, 2, 3, 4, 5 or 6 puffs in a single doses (inhalation events), respectively.

Collection of blood samples for determination of esketamine and esnorketamine concentration and calculation of pharmacokinetic parameters was performed for up 24 hours following the start of the test.

The aim of the study was to determine the amount of puffs needed to obtain plasma concentration similar to that sufficient to achieve antidepressant effect as for 0.20 mg/kg 40 minutes intravenous infusion. It can be predicted on the basis of literature data that this corresponds to concentration at 40 min of infusion between about 60 to 100 ng/ml. It was also the aim to determine the number of puffs that allow to avoid a sharp peak of plasma concentration that is considered an important factor inducing adverse psychomimetic and dissociative effects.

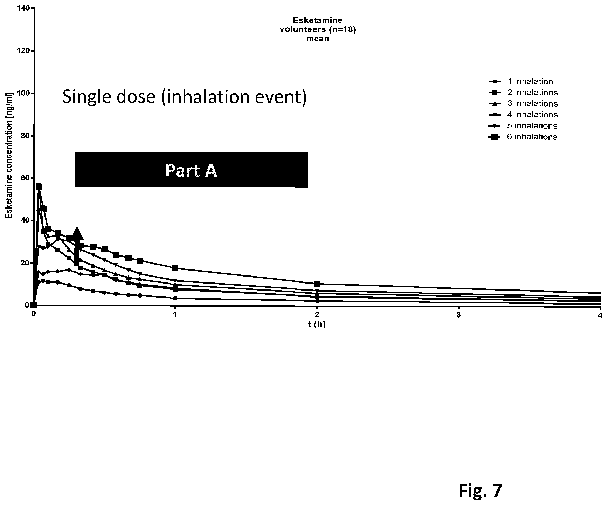

The results of the part A of the test are presented on Figure 7 that shows esketamine plasma concentration over time after administration of various single doses of dry powder composition of Example 2. As it can be seen, the number of puffs that allows to obtain plasma esketamine concentration sufficient for antidepressant effect and without sharp peak of said concentration was determined to be 1 to 4 puffs, corresponding to 4 to 16 mg of esketamine free base nominal dose.

Therefore, a single dose (inhalation event) consisting of 1 to 4 puffs was selected for the next Part B of the test.

In part B of the study the composition of Example 2 was administered to 12 healthy volunteer subjects divided into 4 cohorts in four different single doses each cohort (i.e. each single dose consisting of 1, 2, 3 or 4 puffs, respectively) in one day in the administration sequence consisting of three administrations of single dose (inhalation event) in the period of 30 minutes, Between inhalation events there were 15 minutes break periods, i.e. first single dose was administered at 0 min., second single dose was administered at 15 min, and third single dose was administered at 30 min.

The aim of Part B was to investigate pharmacokinetic properties of esketamine following different dosing schemes in healthy subjects and determine the scheme that enables achievement of the appropriate plasma concentration over time to mimic the 40-minute intravenous infusion (part B),

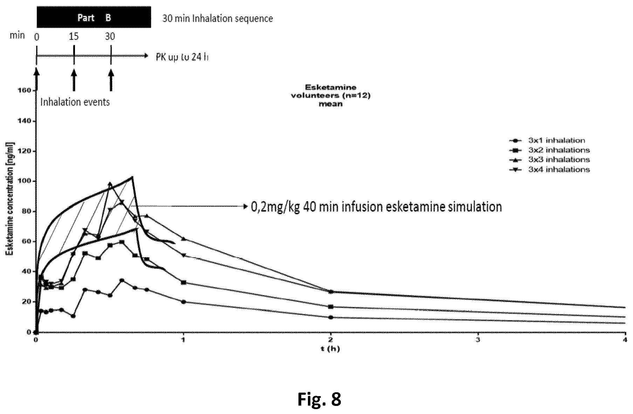

The results of the of the part B of the test are presented on Figure 8 that shows esketamine plasma concentration over time after administration of various single doses of dry powder composition of Example 2 in a sequence of 3 administrations of single doses during 30 minutes. Figure 8 shows also (the area between two bold black lines) a simulation of esketamine plasma concentration after 0.2 mg/kg 40 minutes i.v. infusion.

As it can be seen form Fig. 8, sequence of administration of 3 single doses consisting of 3 or 4 puffs allowed to obtain plasma concentration profile mimicking quite well esketamine intravenous infusion at the level corresponding to antidepressant effect.

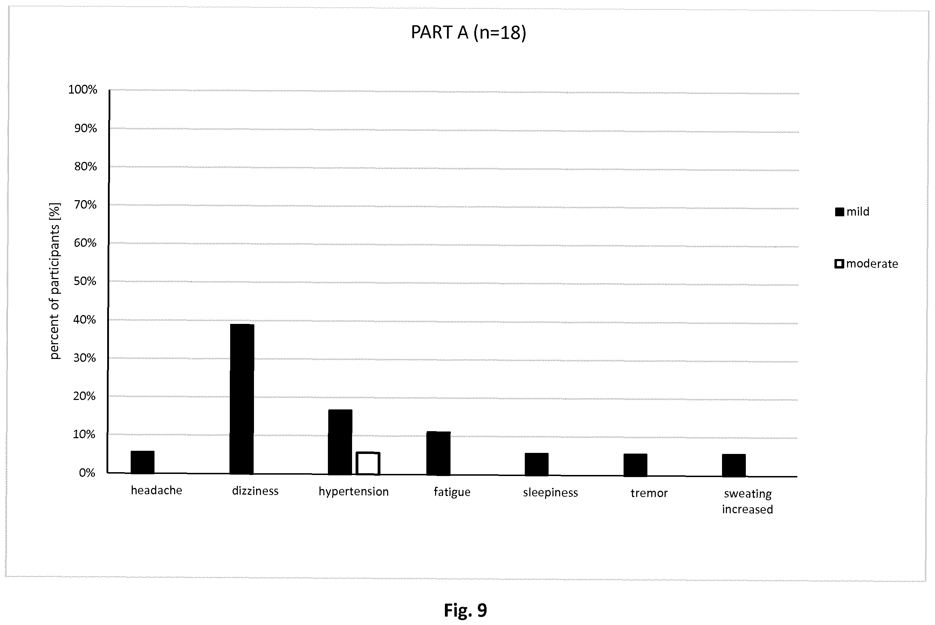

Both in Part A and Part B of the study the adverse effects were monitored and assessed by a psychiatrist. The summary of the adverse effects is presented in Fig. 9. As can be seen, no serious effects were observed, all adverse effects being assessed as mild, occasionally moderate. Psychomimetic effects were transient, lasting up to 30 minutes following administration. There were no discontinuations due to adverse effects or toxicity.

The above shows that pulmonary administration of esketamine, i.e. directly to the lungs is a promising way of treating depression, in particular TRD, by convenient self-administration by a patient. Plasma concentration profile is quite smooth, consistent with a target profile and safe for chronic administration.

The system for electronically supervised administration of a pharmaceutical composition with an inhaler according to the present invention is disclosed in a non-limiting embodiment relating to a dry powder inhaler used in therapy of a drug resistant depression.

The system for electronically supervised parenteral administration of a pharmaceutical composition with an inhaler according to the present invention comprises a digital communications means, a control terminal for an authorised entity, and an inhaler for administration of a pharmaceutical composition. Preferably, an inhaler may be provided with sensors for measuring at least one physical property characterizing administration process from a perspective of an inside of the inhaler, and further a system may comprise a processing station that is adapted to convert the measured physical value and convert it into a quality measure of the administration process.

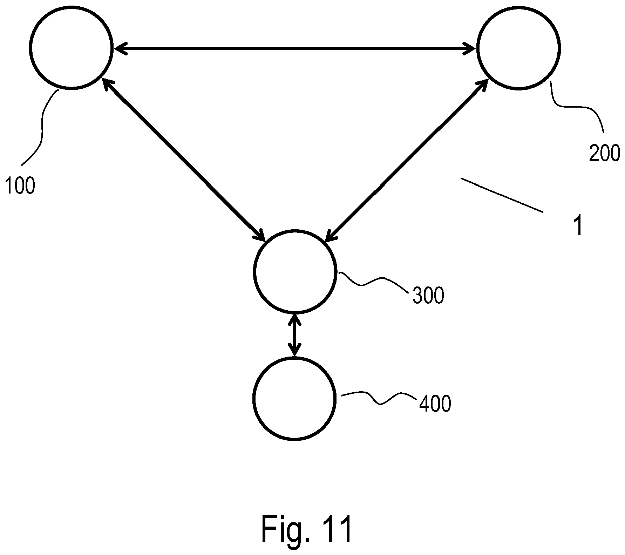

In the embodiment of the system presented on Fig. 11 a system **1** comprises several nodes: a control terminal 100 for an authorised entity, a processing station **200** and an inhaler **400** for administration of a pharmaceutical composition, along with a patient's mobile device **300.** Communications means are depicted symbolically by arrows which are elements of the communication system capable of establishing a communication link between the system nodes, preferably a secure encrypted communication link with a use of TLS/SSL encryption protocols.

A communications means might be any standard communications means of digital communication known in the art capable of transmitting a message frames between nodes of the system, this includes cable, wireless, ground or satellite communications systems supporting Internet communications protocols TCP/IP. The communication means covers also near field communications systems, like NFC, Bluetooth, etc. These are particularly suitable for establishing a communication link between a patient's mobile device **300** and the inhaler **400.**

The mobile device **300** is a mobile phone, tablet, electronic watch, band or any other handheld or wearable device with a user interface, memory, processing means and communications means. The mobile device needs to be provided with an unique identification data allowing to distinguish this device from other devices.

In the first embodiment a control terminal **100** is a computer terminal provided with a user interface allowing interaction of the authorised entity with the control terminal **100.** The authorised entity might be a physician that has selected particular treatment to the patient that needs to be implemented with a use of the inhaler **400** according to the invention being part of the system **1.** However, the authorised entity might also be an institution or a number of institutions within the local health care system. For example the authorised entity may comprise of a physician selecting the treatment for a patient by issuing a regular prescription, a pharmacist working in a drug store who is going to issue the inhaler to the patient or a pharmaceutical company manufacturing inhalers loaded with the pharmaceutical composition. The common feature of the authorised entity is that at least one person within the entity has an authorisation to qualify the particular identified patient for therapy with a use of the pharmaceutical composition distributed within the inhaler **400,** and there is at least one terminal that is generating a control signal **5** with an administration scheme **11** and an authorisation token **12** for assigning to a patient's mobile device **300.** Preferably the authorisation entity may be one person, e.g. a physician issuing a prescription, however a distributed authorisation entity of the functionality described above is equally feasible.

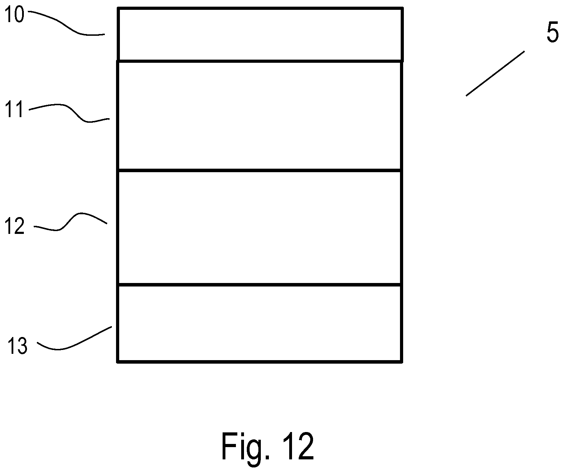

Fig. 12 shows the control signal **5,** the control signal **5** is generated when the administration scheme **11** is selected for the patient. The control signal **5** comprises a unique identification data **10** of the control signal, with an administration scheme **11** and an authorisation token **12** for assigning to a patient's mobile device, and a security block **13.** By generating both the data corresponding to an administration scheme **11** and the authorisation token **12** for the mobile device **300,** the system **1** is simplified because the authorised entity is required to communicate only with the mobile device **300.** It is not necessary for the authorised entity to communicate additionally with the medical device **400** directly. By providing the data corresponding to an administration scheme **11** and the authorisation token **12** in the same signal, the system **1** is simplified because only one signal **5** is required.

The identification data **10** may be an ID code of the control signal **5** or a signal ID header comprising a time stamp, a serial number of the control signal, a prescription number etc. The primary function of the identification block **10** is to uniquely identify an event of generation of a control signal **5.**

The administration scheme **11** is part of the control signal **5,** that identifies the pharmaceutical composition and administration parameters prescribed for the patient. The administration scheme **11** might be simply an identifier of the approved standard therapy, or set of data indicating the pharmaceutical composition, administration regime, dose etc, or it may be a set of data that identifies the pharmaceutical composition while the administration scheme is personalized according to the therapeutic needs of the patient. Preferably the control terminal **100** is provided with a cross checking function that is cross checking the personalized parameters of the administration scheme with approved ranges.

The authorisation token **12** is part of the control signal that is unique to the control signal and represents the approval to use the prescribed pharmaceutical composition by the patient. This might be a serial number or hash unique for the approval granted. The authorisation token might be a pure electronic code, or might have a physical form of a sticker or tag provided with an insignia readable by the mobile devices, e.g. 3D code, 2D code, QR code, NFC tag. Hence, the authorisation token **12** may be provided separately from the control signal **5.**

The security block **13** comprises data allowing verification of the integrity of the control signal **5,** and allowing to identify the authorised entity that issued the control signal **5.** This can be a block comprising digital certificate of the authorised authority that generated the control signal **5,** and a hash block generated for the control signal **5** with a use of the digital certificate. The security block may implement any feasible integrity control system.

Preferably the control signal **5** is encrypted, and the communication means implements secure communication channels as, for example, with the use of the known protocols and encryption schemes.

The control signal **5** might be a single data packet/message or a collection of independent packets or messages linked in a way providing its integrity and functionality as described above. For example the administration scheme **11** might be one of a number of different standard administration schemes stored in the memory of the inhaler **400** with their identifiers, while the control signal **5** generated by the physician comprises only an identifier pointing to the administration schemes to be applied. Alternatively, a control signal **5** is generated by a pharmacist based on a prescription issued by a physician, and includes an identifier of the administration scheme and an authorisation token issued by the pharmaceuticals company being responsible for manufacturing the pharmaceutical composition within the inhaler.

When a physician qualifies a patient for the treatment with a pharmaceutical composition via an inhaler according to the invention the administration scheme is selected. Thus, the first element of the control signal **5** is being created. At this time the physician can issue a prescription for the inhaler **400** to be collected at the pharmacy, or alternatively, it can provide the patient with the inhaler **400.** The moment the inhaler **400** is provided to the patient, the authorisation token **12** is assigned to the patient's mobile device **300.** Alternatively, the authorisation token **12** is assigned to the mobile device **300** of a patient when the prescription for an inhaler **400** is issued by the physician.

Assignment of the authorisation token **12** to the mobile device **300** comprises a step of transferring the authorisation token **12** to the memory of the mobile device **300.** This transfer may take several forms, e.g. by scanning a QR code with an authorisation token generated by the pharmaceutical company by the camera of the mobile device that is further decoded by the software of the mobile device **300** and stored in the memory of the mobile device **300.** Further, assignment of the authorisation token **12** to the mobile device **300** comprises a step of transferring the authorisation token **12** along with an identification data of the mobile device **300** to the authorised entity that issued a control signal **5.**

In order to perform the assignment steps the patient's mobile device **300** needs to be provided with a software application allowing transferring the authorisation token **12** to the memory of the mobile device and further communicating the authorisation token **12** with the mobile device **300** identification data to the processing station **200** of the authorised entity responsible for generating a control signal **5.** The processing station **200** assigns the authorisation token **12** to the mobile device **300** using a mobile device's identification data, linking the mobile device **300** with the authorisation token **12.** The processing station **200** generates a confirmation of assigning a mobile device **300** with the authorisation token **12** and sends back this confirmation to the mobile device **300.** The mobile device **300** is also adapted to receive from a processing station **200** a confirmation of assigning the authorisation token **12** to the mobile device **300.**

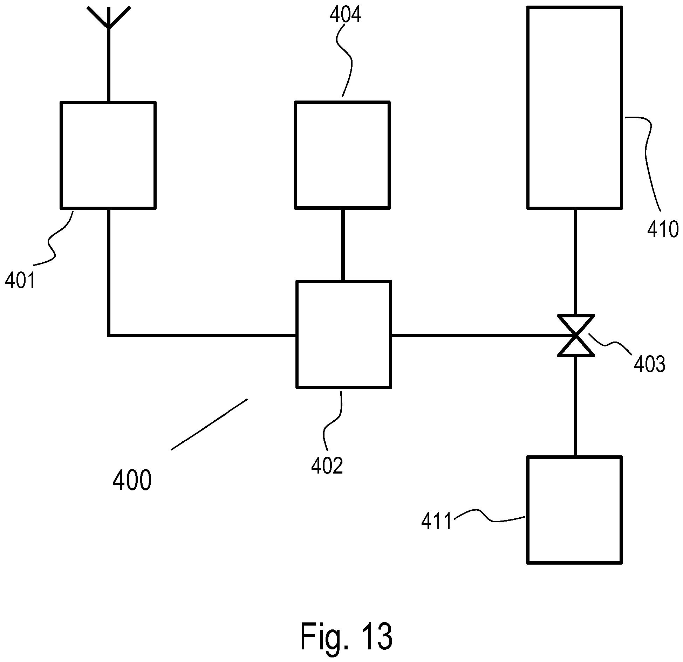

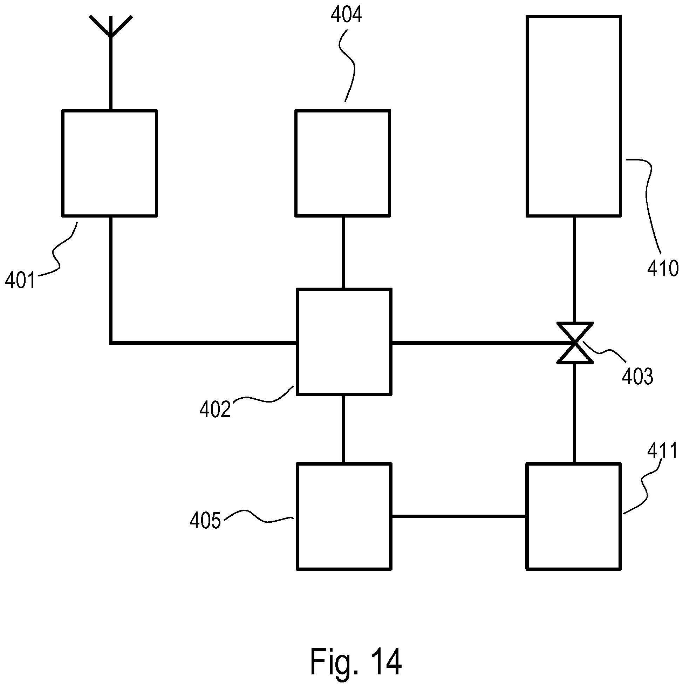

The inhaler **400** according to the present invention as presented on Fig. 13 comprises a communications unit **401,** a processing unit **402** comprising a clock, a controlled blocking unit **403,** a memory unit **404,** a pharmaceutical composition storage **410** and a pharmaceutical composition administration unit **411.** As shown on Fig. 14 the inhaler **400** preferably is provided with a measurement unit **405** that is provided with a sensor adapted to measure a physical property of the administration process of the pharmaceutical composition. The sensor unit **405** may comprise a microphone, and the measured physical property may be an amplitude of a sound wave. The microphone may be placed inside the mixing chamber where a dry powder pharmaceutical composition is mixed with air during the inhalation.

The inhaler **400** is a device preloaded with a pharmaceutical composition and adapted to administer the pharmaceutical composition in a predetermined doses. Preferably the inhaler **400** is a sealed inhaler. It means it does not allow to refill or open or modify the content of the storage unit **410** for a pharmaceutical composition. Alternatively, the inhaler **400** is adapted to allow replacing the content of the storage unit **410** in a controlled manner.

A patient who received the inhaler **400** and was assigned the authorisation token **12** to the patient's mobile device **300,** registers in the inhaler **400** the authorisation token **12** assigned to the mobile device **300.**

Registration of the authorisation token **12** assigned to the mobile device **300** means transferring the authorisation token **12** assigned to the patient's mobile device **300** into the memory of the inhaler **400.** Alternatively, the inhaler **400** registers a confirmation that the authorisation token **12** has been assigned to the patient's mobile device **300.** This can be done via the communications means establishing a communication channel between the inhaler **400** and the mobile device **300.** Preferably the communication channel is a near field or close range communication channel or the communication channel enables the distance measurement between the mobile device **300** and the inhaler **400.**

In response to registration of the authorisation token **12** assigned to the mobile device **300** with the inhaler **400,** the inhaler **400** processes the administration scheme **11.** Processing the administration scheme means the inhaler **400** makes the administration scheme **11** an active administration scheme and allows administration of the doses of a pharmaceutical composition in a time windows indicated by the administration scheme **11.** In order to follow the administration schemes the inhaler **400** is provided with a controlled blocking means **403** that effectively blocks the transfer of a dose of the pharmaceutical composition from the storage **410** to the administration unit **411,** and upon receiving a control signal from the processing unit **402** allows the transfer of a dose of the pharmaceutical composition from the storage **410** to the administration unit **411.**

The blocking means **403** comprises for an example a valve, pin, bolt, relay, key, normally closed switch or any form of actuator that is in a blocking position that blocks the transfer of a dose of the pharmaceutical composition from the storage **410** to the administration unit **411,** and may be positioned in an open position allowing administration of the pharmaceutical composition in response to a control signal from the control unit **402.** The blocking means **403** is normally closed (normally closed type) and opens only according to the active administration scheme **12.** The controlled blocking means **403** may comprise a drive unit and active actuating element that blocks the transfer of a dose of the dry powder pharmaceutical composition from the storage **410** to the administration unit **411.** The actuating element in a blocking state may block the transfer of a dose of the pharmaceutical composition from the storage **410** to the administration unit **411,** and in an open position allows administration of the pharmaceutical composition in response to a control signal from the control unit **402.** Upon receiving a control signal from the processing unit **402** the actuating element may move into an open state and allow administration of the pharmaceutical composition.

Further, the inhaler **400** is adapted in a such way that a controlled blocking means **403** allows the administration of a pharmaceutical composition stored in the storage **410** only with compliance with the administration scheme **11** and in the presence of the patient's mobile device **300** with the authorisation token **12** assigned thereto. The presence of the patient's mobile device **300** shall be understood as the mobile device **300** being in a proximity of the inhaler **400,** i.e. the distance between these two devices being less than 10 meters, preferably less than 5 m, most preferably less than 2 m. By providing the mobile device **300** with the authorisation token **12** and requiring the mobile device **300** to be present, the system **1** is more secure than systems that require the patient to authenticate himself directly on the medical device **400.** This is because the pharmaceutical composition can be administered only when the mobile device **300** is present, rather than only requiring the presence of the medical device **400.** As a result, the system **1** is secure even if an unauthorised person has the medical device **400** and the patient's passcode, for example.

Therefore, the inhaler **400** is adapted to cross-check the presence of the mobile device **300** in the proximity of the inhaler **400.** This can be achieved by a number of methods, for example using a close range communication means. In such solution a lack of communication connection between the inhaler **400** and the mobile device **300** is understood as being out of range position of the two, hence, the distance between the two devices is larger than expected.

Alternatively, the inhaler **400** is provided with a range finder that actively or passively determines the distance between the inhaler **400** and the mobile device **300,** for example a laser rangefinder, acoustic range finder, time delay measurement system, phase-shift rangefinders, etc.

If the distance between the mobile device **300** and the inhaler **400** is larger than the prescribed limit, this makes one of the conditions for administration of the pharmaceutical composition missing, therefore, the processing unit **402** is not sending a control signal to the controlled blocking means **403,** this does not allow the administration of the pharmaceutical composition. The administration of the pharmaceutical composition is possible only when both conditions are fulfilled, i.e.:
a) the clock of the control unit **402** indicates the time fall within the time window of administration of a dose according to the active administration scheme **11,** and
b) the patient's mobile device **300** with the assigned authorisation token **12** is in the proximity of the inhaler **400.**

System **1** by combining these two conditions provides an effective way to control abuse and misuse of the pharmaceutical composition. First of all, assigning the authorisation token **12** to the patient's mobile device **300** guaranties the inhaler **400** can be activated only by the authorised person. This is due to the fact of a new phenomenon observed which strongly binds the person with a mobile device on an emotional level.

As shown on Fig. 14 the inhaler **400** preferably is provided with a measurement unit **405** that is provided with a sensor adapted to measure a physical property of the administration process of the pharmaceutical composition. The physical property of the pharmaceutical composition administration process measured within the inhaler **400** during the administration process is an air pressure, sound intensity, vibration magnitude or any combination of such physical properties. This measurement of the internal physical process that happens inside the inhaler allows to get information confirming the dose has been administered and information about the quality of the administration process i.e. was this process a correct or a failed one. The information on the fact the dose has been administered and on the quality of the administration process is valuable information that can assess the compliance of the patient with the administration scheme and quality of the performance of the patient when the administration process requires active participation of the patient as this is a case in dry powder inhalators when the pharmaceutical composition is excited by air inhaled by the patient. The quality of such process depends on the airflow generated by the patient when taking a dose.

The data gathered during the administration process are communicated to the processing station **200** of the authorised entity. The processing station **200** is adapted to convert the convert data representing the measured physical property into a quality measure of the administration process. Preferably the quality measure is a value of an abstract index such as 0 or 1, or a grade composed of natural number between 0 and 10, or any other valued measure that is capable of representing the quality of the administration process. This value can be calculated based on a function based on a single variable or multivariable, differential equation or set of equation, fed by measured values of the physical property or properties in time domain, frequency domain or in any suitable transform formed.

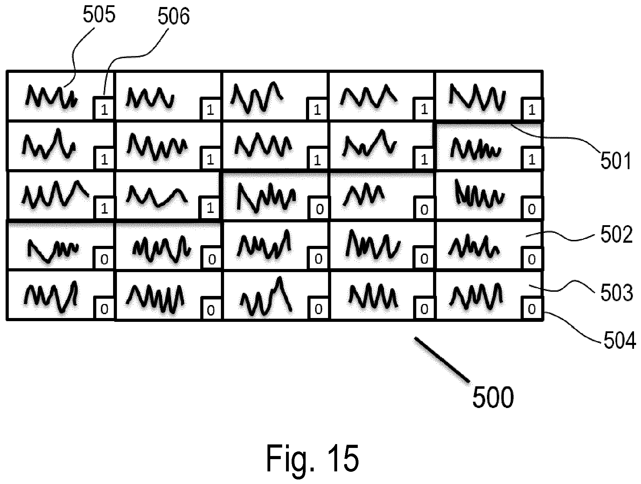

Preferably the value of the quality measure is selected based on a heuristic observations that allow to assign the value of quality measure to the pattern being a representation of the measured physical property in time domain or frequency domain. Preferably within a process of heuristic observations a ranking matrix **500** is produced as shown on Fig. 15. The ranking matrix **500** comprises a set of fields comprising patterns **420** representing measured values of the physical property as explained above. The fields in the ranking matrix are organised into two areas divided by a solid border **501,** each pattern received having an assigned quality measure of the administration process. An organisation of fields in the ranking matrix into two separate areas with different quality values is not required, the ranking matrix **500** may be scattered and fields need not to create continued areas with solid boundaries. The ranking matrix **500** works as long as each and every field of the matrix **502** with a specific pattern has an assigned quality value. For example field **503** has an assigned quality value **504** of 0 and the filed **505** has an assigned quality value **506** of 1.

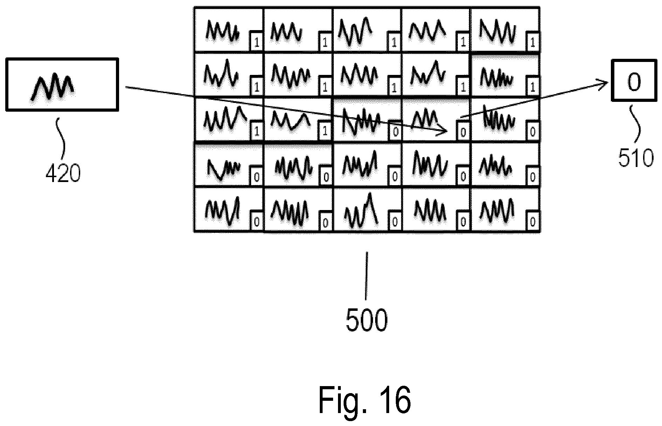

Fig. 16 shows the process of obtaining a quality measure value from the ranking matrix **500** for a pattern **420** measured within the inhaler **400.** The pattern **420** representing measured values of the physical property is compared with patterns in fields of the ranking matrix **500** in order to establish a measure of similarity. The measure of similarity is selected using known methods of establishing a similarity, like mean square error, least squares etc. The pattern in the ranking matrix **500** for which the best similarity measure is identified, for example for which the mean square error is the smallest, is considered the best fit and the quality value **510** assigned to this field is returned in the result of this process. This process can be described as best fit rule for selecting a value of a quality measure.

The processing station **200** is adapted to communicate the value **510** of the quality measure of the administration process to the control terminal **100** when the control terminal **100** is operated by the physician that qualified the patient for treatment with a pharmaceutical composition. The control terminal is adapted to present the received quality measure of the pharmaceutical composition administration process to the physician or authorised entity using the user interface. This feed-back loop allows to asses a compliance of the patient with an administration scheme. Such information can be used to amend the administration scheme of the present pharmaceutical composition or switch to a different pharmaceutical composition if a current treatment lacks of effect though the administration of the pharmaceutical composition was correct.

The processing station **200** preferably returns the value **510** of the quality measure of the administration process to the patient's mobile device **300,** this improves the self-control of the patient and supports the patient's motivation by confronting the patient with a quality measure. All these factors improve the compliance of the patient with an administration scheme and have a positive therapeutic effect.

Preferably the processing station **200** is selected from a group of processing devices comprising mobile phone, personal computer, mainframe computer, cloud computing system or any combination of such computing devices with communication, processing and storage capabilities suitable for the processing digital signal and handle database operation, with a controlled access. As described above the processing station **200** performs two functions within the system. The processing station **200** is assigning the patient's mobile devices with an authorisation tokens, and further the processing station **200** is converting the measured physical property into a quality measure of a value representing quality of the pharmaceutical composition's administration process/event.

Having in place the system for electronically supervised parenteral administration of a pharmaceutical composition a new method for treatment of a disease in a patient in need thereof is obtained. The method comprising parenteral self-administration of pharmaceutical composition by said patient via medical device in a remotely dictated and controlled manner in accordance with a self-administration scheme **11** prescribed by the attending physician, in the presence of a patient's mobile device **300** with an authorisation token **12** assigned thereto, wherein said medical device is operated in compliance with the self-administration scheme **11** via a controlled blocking means **403** adapted to allow administration of a pharmaceutical composition only with compliance with the administration scheme **11** in the presence of a patient's mobile device **300** with the authorisation token 12 assigned thereto.

Administration process is allowed by the controlled blocking means **403** only with compliance with the administration scheme and in the presence of a patient's mobile device **300** with the authorisation token **12** assigned thereto. This two levels of control delegate the supervision over the administration of the pharmaceutical composition to the electronic system.

As the administration is protected by the controlled blocking means **403** against misuse or abuse by the patient or a third person it can be safely applied to a rage of substances that in the past required personal supervision of the qualified personnel.

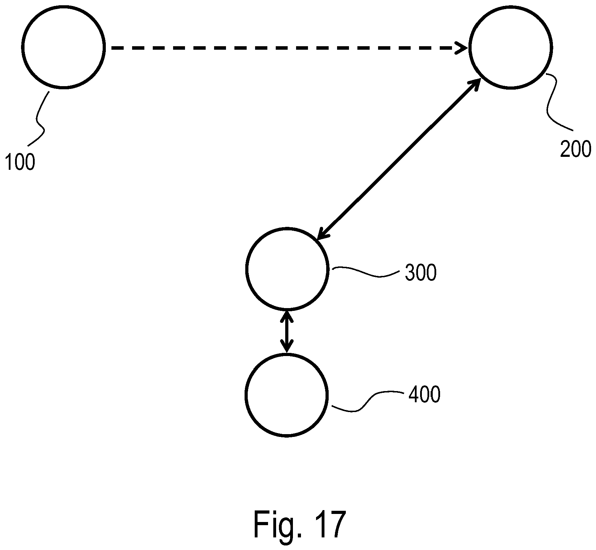

In the second embodiment as shown on Fig. 17 the system **1** comprises a processing station **200** adapted to transmit the control signal to the inhaler, and wherein the inhaler receives the control signal with the administration scheme from the processing station in response to registration of the authorisation token with the inhaler. This alternative route might be more convenient in the countries where the physicians do not have a computer or the communication networks do not offer stability required to generate the authorisation token when qualifying the patient for the treatment. This scheme can be also applied when the physicians are needed to be released from administrative tasks.

The second embodiment of the system provides the same level of security and is equally robust to third party interreference. In the second embodiment the processing station **200** takes over a communication function from the control station **100.** A communication channel between the control station **100** and processing station **200** may be of a different character that the communication channel established between the processing station **200** and the mobile device **300.**

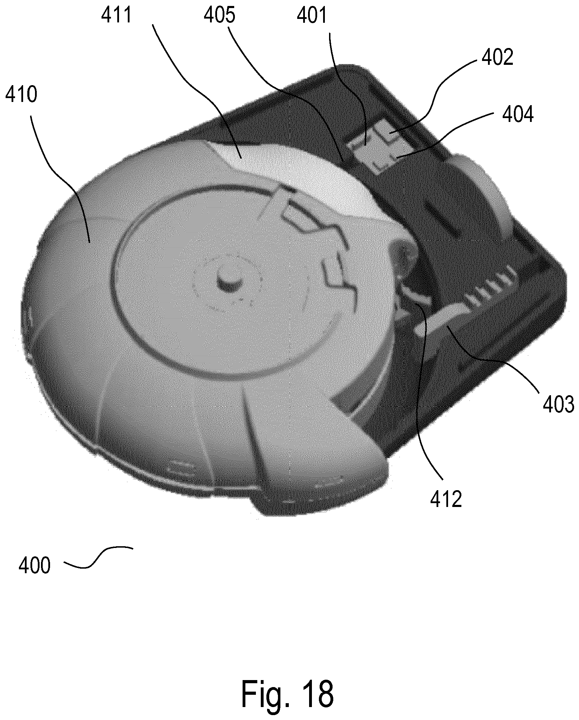

Fig. 18 shows a another embodiment of the inhaler **400** according to the invention for use in treatment of depression. This inhaler comprises a storage **410,** that holds a blister of a single doses of a dry powder pharmaceutical composition. In this embodiment a pharmaceutical composition is an esketamine in a form of a dry powder composition. Inhaler **400** comprises also an administration unit **411.** The administration unit **411** is provided with a mixing chamber and airflow channels. Administration unit **411** is controlled by the loading handle **412,** pulling the handle **412** releases the dose of a dry powder pharmaceutical composition into the mixing chamber of the administration unit **411.** When the dose of the dry powder pharmaceutical composition is in the mixing chamber, the inhaler **400** is ready to use by the patient.

Fig. 18 shows the inhaler **400** according to the invention provided with a control module comprising a communication means **401,** control means **402,** blocking means **403,** memory **404,** and preferably a measurement unit **405.**

Fig. 18 shows the inhaler **400** in the closed configuration suitable for storage or transportation. In this configuration the inhaler **400** does not allow administration of the pharmaceutical composition. In order to open the inhaler **400** into the open configuration, the storage **410** and administration unit **411** need to be rotated out of the control module. In the closed configuration the administration unit **411** is covered by the control module that blocks access to the administration unit **411.** Rotating the storage **410** and administration unit **411** out of the control module, exposes administration unit **411** to the patient. In the closed configuration the blocking means **403** objects to the rotation of the storage **410** with administration unit **411.** The blocking means **403** in the form of a bolt is extending into a channel in which the handle **412** travels when the storage **410** and administration unit **411** are rotated during the conversion of the inhaler **400** from the closed configuration into the open configuration. The blocking means **403** effectively blocks the turn of the storage **410** and administration unit **411** in this way blocking means **403** does not allow the inhaler to be converted into the open configuration thus, does not allow to administer the pharmaceutical composition.

The control module of the inhaler **400** comprises communication means **401,** control means **402,** blocking means **403,** memory **404,** and preferably a measurement unit **405.** The power source and drive unit for actuating the blocking means (not depicted) are also within the control module.

The control means **402** of the inhaler **400** processes the administration scheme **11** in response to registration in the inhaler **400** of the authorisation token **12** assigned to the patient's mobile device **300,** while the controlled blocking means **403** allows administration of the dry powder pharmaceutical composition only with compliance with the administration scheme **11** in the presence of a patient's mobile device **300** with the authorisation token **12** assigned thereto.

Registration of the authorisation token **12** assigned to the inhaler **300** means transferring the authorisation token **12** assigned to the patient's mobile device **300** into the memory **404** of the inhaler **400.** Alternatively, the inhaler **400** registers a confirmation that the authorisation token **12** has been assigned to the patient's mobile device **300.** This can be done via the communications means **401** establishing a communication channel between the inhaler **400** and the mobile device **300.** Preferably the communication channel is a near field or close range communication channel or the communication channel enables the distance measurement between the mobile device **300** and the inhaler **400,** for example NFC or Bluetooth.

The administration scheme **11** in this embodiment is pre-stored in the memory **404** of the inhaler **400.** However, it can be transmitted along with the authorisation token **12** and then stored in the memory **404** of the inhaler **400.**

The control means **402** provided with administration scheme **11** determines the time slots when the inhaler **400** can be converted from the closed configuration into the open configuration. As the second level of protection against abuse and misuse of the pharmaceutical composition in this case esketamine, the control unit **402** checks if a patient's mobile device **300** with the authorisation token **12** assigned thereto is present near the inhaler **400.**

Therefore, the inhaler **400** cross-checks the presence of the mobile device **300** with the authorisation token **12** assigned thereto, in the proximity of the inhaler **400.** This is done by using a close range communication means. The Lack of communication connection between the inhaler **400** and the mobile device **300** is understood as being out of range position of the two, hence, the distance between the two devices is larger than expected.

Having the two conditions fullfield at the same time the control unit **402** is providing a control signal to the drive unit to withdraw the blocking means **403** from the channel in which the handle **412** travels, allowing this way to convert the inhaler **400** from the closed configuration into the open configuration.

Having in place the system **1** for electronically supervised administration of a pharmaceutical composition a method for treatment of depression in a patient in need thereof, one can implement the method comprising self-administration of ketamine or its pharmaceutically acceptable salt by said patient by pulmonary route as dry powder inhalable pharmaceutical formulation via an inhaler **400** in a remotely dictated and controlled manner in accordance with an administration scheme **11** prescribed by the attending physician, in the presence of a patient's mobile device **300** with the authorisation token **12** assigned thereto. In the method of use said inhaler **400** is operated in compliance with the administration scheme **11** via a controlled blocking means **403** adapted to allow administration of a pharmaceutical composition only with compliance with the administration scheme **11** in the presence of a patient's mobile device **300** with the authorisation token **12** assigned thereto. The inhaler **400** may comprise ketamine or its pharmaceutically acceptable salt for use in a method of treatment of depression, wherein ketamine or its pharmaceutically acceptable salt is administered by the pulmonary route as a dry powder pharmaceutical composition. The pharmaceutically acceptable salt may be hydrochloride. The ketamine may be esketamine hydrochloride.

The composition may comprise from 2 mg to 100 mg of micronized ketamine calculated as a free base per nominal unit dose. The composition may comprise from 2 mg to 40 mg of micronized ketamine calculated as a free base per nominal unit dose. The composition may comprise 4 mg of micronized esketamine calculated as a free base per nominal unit dose. The composition may comprise one or more additives selected from the group consisting of a carbohydrate bulking agent in the amount of 30 to 95% by weight and a stabilizing agent in the amount of 0.2 - 3% by weight, with respect to the total weight of the composition. The composition may comprise ketamine having median particle diameter d50 of 1 - 10 µm, d10 of 0.2 - 5 µm and d90 of 3 - 35 µm, as measured by laser diffraction technique. The inhaler may be adapted to provide emitted dose of at least 1.0 mg of ketamine calculated as a free base, corresponding to 1.2 mg of ketamine hydrochloride. The fraction 5 of the emitted dose delivered to the lungs may be at least 40%.

The composition for administration via pulmonary route may be comprised in a blister with a plurality of individual nominal unit doses premetered and individually sealed. The composition for administration via pulmonary route may be comprised in a capsule with a single nominal unit dose. The composition for administration via pulmonary route may be comprised in a multi-dose powder reservoir.

The administration scheme **11** may provide a self-administration by a patient by inhalation of a dry powder ketamine composition or formulation in a sequence of administrations consisting of multiple single doses, for example such as a sequence of at least 3 single doses, each single dose consisting of multiple puffs, such as 1, 2, 3 or 4 puffs, preferably 3 or 4 puffs, said sequences being separated from each other by a break period without any inhalation. The administration scheme **11** may comprise the sequence of esketamine three single doses consisting of 3 or 4 puffs in a period of 30 minutes, single doses being separated by a break periods of 15 minutes, wherein each puff corresponds to esketamine nominal dose of 4 mg in the dry powder composition or formulation.

## Claims

1. An inhaler (400) for electronically supervised parenteral administration of a dry powder pharmaceutical composition comprising:
storage means (410) for the pharmaceutical composition in a form of a dry powder;
administration means (411) for administration of the pharmaceutical composition, provided with a mixing chamber and airflow channels;
memory (404) and processing means (402);
communication means (401);
controlled blocking means (403) for blocking administration of the pharmaceutical composition, wherein
the inhaler (400) is adapted
to be registered with an authorisation token (12) assigned to a mobile device (300),
to receive data corresponding to an administration scheme (11), the inhaler (400) is further adapted
to determine whether in the proximity of the inhaler (400) there is present the mobile device (300) with the registered authorisation token (12) assigned thereto by an authorised entity (100, 200) entitled to qualify for therapy with a use of the pharmaceutical composition with at least one terminal that is generating a control signal (5) with an administration scheme (11) and the authorisation token (12), and
in response to registration of the authorisation token (12) assigned to the mobile device (300) in the inhaler (400), the inhaler is configured to process the administration scheme (11), by making the administration scheme (11) an active administration scheme and allowing administration of the doses of a pharmaceutical composition in a time window indicated by the administration scheme (11), such that the inhaler controls the controlled blocking means (403) so as to allow administration of the pharmaceutical composition only with compliance with the administration scheme (11), and in the presence of the mobile device (300) with the registered authorisation token (12) assigned thereto by the authorised entity (100, 200).

2. The inhaler according to claim 1 wherein the inhaler is adapted to be registered with the authorisation token (12) assigned to the mobile device (300) by transferring the authorisation token (12) assigned to the patient's mobile device (300) into the memory (404) of the inhaler (400).

3. The inhaler according to claim 1 or 2 wherein the inhaler (400) is further
comprising a sensor unit (405) and is adapted to
measure via the sensor unit (405) at least one physical property of the dry powder pharmaceutical composition administration process within the inhaler (400) during an administration process and
communicate the measured physical property to the mobile device (300).

4. The inhaler according to claim 3 wherein the physical property of the pharmaceutical composition administration process measured within the inhaler (400) during an administration process is an air pressure, sound intensity, vibration magnitude or any combination of such physical properties.

5. The inhaler according to any of the claims 3 or 4 wherein the sensor unit (405) comprises a microphone, and the measured physical property is an amplitude of a sound wave.

6. The inhaler according to any of the claims from 3 to 5 wherein the sensor unit (405) is placed inside the mixing chamber where a dry powder pharmaceutical composition is mixed with air during the inhalation.

7. The inhaler according to any of the claims from 1 to 6 wherein the controlled blocking means (403) comprises a drive unit and an active actuating element that blocks the transfer of a dose of the dry powder pharmaceutical composition from the storage (410) to the administration means (411).

8. The inhaler according to any of claims from 1 to 7 wherein, the blocking means (403) comprising actuating element and actuating element in a blocking state blocks the transfer of a dose of the pharmaceutical composition from the storage (410) to the administration means (411), and in an open position allows administration of the pharmaceutical composition in response to a control signal from the processing means (402).

9. The inhaler according to any of claims from 1 to 8 wherein the blocking means (403) comprises an element selected from a group comprising: a valve, pin, bolt, relay, key, normally closed switch.

10. The inhaler according to any of the proceeding claims from 1 to 9 wherein the storage means (410) comprising a dry powder pharmaceutical composition comprising ketamine or its pharmaceutically acceptable salt for use in a method of treatment of depression, by direct administration to the lungs via pulmonary route.

11. The inhaler according to any of the proceeding claims from 1 to 9 wherein the storage means (410) comprising ketamine or its pharmaceutically acceptable salt for use in a method of treatment of depression, wherein ketamine or its pharmaceutically acceptable salt is administered by pulmonary route as a dry powder pharmaceutical composition.

12. The inhaler according to any of claims 10 or 11 wherein pharmaceutically acceptable salt is hydrochloride.

13. The inhaler according to any of claims from 10 to 12 wherein ketamine is esketamine hydrochloride.

14. The inhaler according to any of claims from 10 to 13 wherein the composition comprises from 2 mg to 100 mg of micronized ketamine calculated as a free base per nominal unit dose.

15. The inhaler according to claim 14 wherein composition comprises from 2 mg to 40 mg of micronized ketamine calculated as a free base per nominal unit dose.

## Patentansprüche

1. Inhalator (400) zur elektronisch überwachten parenteralen Verabreichung einet pharmazeutischen Zusammensetzung in Form von Trockenpulver, der Folgendes umfasst:
Speicher (410) für die pharmazeutische Zusammensetzung in Form von Trockenpulver;
Verabreichungsmittel (411) für die Verabreichung der pharmazeutischen Zusammensetzung,
mit einer Mischkammer und Luftstromkanälen;
einem Speicher (404) und Verarbeitungsmittel (402);
einem Kommunikationsmittel (401);
einem kontrollierte Blockiermittel (403) zur Blockierung der Verabreichung der pharmazeutischen Zusammensetzung,
wobei
der Inhalator (400) so angepasst ist,
dass er mit einem Berechtigungs-Token (12) registriert werden kann, der einem mobilen Gerät (300) zugeordnet ist,
um Daten zu empfangen, die einem Verabreichungsschema (11) entsprechen, und der Inhalator (400) ferner dazu geeignet ist,
festzustellen, ob sich in der Nähe des Inhalators (400) das mobile Gerät (300) mit dem registrierten Autorisierungs-Token (12) befindet, das ihm von einer autorisierten Einheit (100, 200) zugewiesen wurde, die berechtigt ist, sich für eine Therapie mit einer Verwendung der pharmazeutischen Zusammensetzung mindestens einem Endgerät zu qualifizieren, die ein Steuersignal (5) mit einem Verabreichungsschema (11) und dem Autorisierungs-Token (12) erzeugt, und
als Antwort auf die Registrierung des dem mobilen Gerät (300) zugewiesenen Autorisierungs-Tokens (12) in dem Inhalator (400) der Inhalator so konfiguriert ist, dass er das Verabreichungsschema (11) verarbeitet, indem er das Verabreichungsschema (11) zu einem aktiven Verabreichungsschema macht und die Verabreichung der Dosen einer
pharmazeutischen Zusammensetzung in einem Zeitfenster, das durch das Verabreichungsschema (11) angegeben ist, erlaubt, sodass der Inhalator die gesteuerten Blockiermittel (403) steuert, um die Verabreichung der pharmazeutischen Zusammenssetzung nur unter Einhaltung des Verabreichungsschemas (11) und in Anwesenheit des mobilen Geräts (300) mit dem registrierten Autorisierungs-Token (12), der diesem von der autorisierten Stelle (100, 200) zugewiesen wurde, zu ermöglichen.

2. Der Inhalator nach Anspruch 1, wobei der Inhalator so angepasst ist, dass er mit dem mobilen Gerät (300) zugewiesenen Autorisierungs-Token (12) registriert wird, indem der dem mobilen Gerät (300) des Patienten zugewiesene Autorisierungs-Token (12) in den Speicher (404) des Inhalators (400) übertragen wird.

3. Inhalator nach Anspruch 1 oder 2, wobei der Inhalator (400) ferner eine Sensoreinheit (405) umfasst und so ausgelegt ist, dass
er
über die Sensoreinheit (405) mindestens eine physikalische Eigenschaft des Verabreichungsprozesses der pharmazeutischen Zusammensetzung in Form von Trockenpulver innerhalb des Inhalators (400) während eines Verabreichungsprozesses messen und
die gemessene physikalische Eigenschaft an das mobile Gerät (300) übermitteln kann.

4. Inhalator nach Anspruch 3, wobei die physikalische Eigenschaft des Verabreichungsprozesses der pharmazeutischen Zusammensetzung, die innerhalb des Inhalators (400) während eines Verabreichungsprozesses gemessen wird, ein Luftdruck, eine Schallintensität, eine Vibrationsstärke oder eine beliebige Kombination dieser physikalischen Eigenschaften ist.

5. Inhalator nach einem der Ansprüche 3 oder 4, wobei die Sensoreinheit (405) ein Mikrofon umfasst und die gemessene physikalische Eigenschaft eine Amplitude einer Schallwelle ist.

6. Inhalator nach einem der Ansprüche 3 bis 5, wobei die Sensoreinheit (405) in der Mischkammer angeordnet ist, in der eine pharmazeutische Zusammensetzung in Form von Trockenpulver während der Inhalation mit Luft gemischt wird.

7. Inhalator nach einem der Ansprüche 1 bis 6, wobei das gesteuerte Blockiermittel (403) eine Antriebseinheit und ein aktives Betätigungselement umfasst, das die Übertragung einer Dosis der pharmazeutischen Zusammensetzung in Form von Trockenpulver von der Speichervorrichtung (410) zur Verabreichungsvorrichtung (411) blockiert.

8. Inhalator nach einem der Ansprüche 1 bis 7, wobei das Blockiermittel (403), das ein Betätigungselement und ein Betätigungselement umfasst, in einem Blockierungszustand die Übertragung einer Dosis der pharmazeutischen Zusammensetzung vom Speicher (410) zur Verabreichungsvorrichtung (411) blockiert und in einer offenen Position die Verabreichung der pharmazeutischen Zusammensetzung in Reaktion auf ein Steuersignal vom Verarbeitungsmittel (402) ermöglicht.

9. Inhalator nach einem der Ansprüche 1 bis 8, wobei das Blockiermittel (403) ein Element umfasst, das aus einer Gruppe ausgewählt ist, die Folgendes umfasst: ein Ventil, einen Stift, einen Bolzen, ein Relais, einen Schlüssel, einen normalerweise geschlossenen Schalter.

10. Inhalator nach einer der vorangehenden Ansprüche von 1 bis 9, wobei der Speicher (410) eine pharmazeutische Zusammensetzung in Form von Trockenpulver umfasst, das Ketamin oder sein pharmazeutisch annehmbares Salz zur Verwendung in einem Verfahren zur Behandlung von Depressionen durch direkte Verabreichung an die Lungen über den pulmonalen Weg umfasst.

11. Inhalator nach einer der vorangehenden Ansprüche von 1 bis 9, wobei der Speicher (410) Ketamin oder dessen pharmazeutisch annehmbares Salz zur Verwendung in einem Verfahren zur Behandlung von Depressionen umfasst, wobei Ketamin oder dessen pharmazeutisch annehmbares Salz auf pulmonalem Weg als pharmazeutische Zusammensetzung in Form von Trockenpulver verabreicht wird.

12. Inhalator nach einem der Ansprüche 10 oder 11, wobei das pharmazeutisch akzeptable Salz Hydrochlorid ist.

13. Inhalator nach einem der Ansprüche 10 bis 12, wobei Ketamin Esketaminhydrochlorid ist.

14. Inhalator nach einem der Ansprüche 10 bis 13, wobei die Zusammensetzung 2 mg bis 100 mg mikronisiertes Ketamin, berechnet als freie Base, pro nomineller Einheitsdosis enthält.

15. Inhalator nach Anspruch 14, wobei die Zusammensetzung 2 mg bis 40 mg mikronisiertes Ketamin, berechnet als freie Base, pro nominaler Einheitsdosis umfasst.

## Revendications

1. Un inhalateur (400) pour l'administration par voie parentérale sous surveillance électronique d'une composition pharmaceutique sous forme de poudre sèche comprenant :
un moyen de stockage (410) pour la composition pharmaceutique sous forme de poudre sèche ;
un moyen d'administration (411) pour l'administration de la composition pharmaceutique, doté
d'une chambre de mélange et de canaux de circulation d'air ;
d'une mémoire (404) et d'un moyen de traitement (402) ;
d'un moyen de communication (401) ;
d'un moyen de blocage contrôlé (403) pour bloquer l'administration de la composition
pharmaceutique, dans lequel
l'inhalateur (400) est adapté
pour être enregistré avec un jeton d'autorisation (12) attribué à un appareil mobile (300),
pour recevoir des données correspondant à un schéma d'administration (11), l'inhalateur (400) est en outre adapté
pour déterminer si, à proximité de l'inhalateur (400), se trouve le dispositif mobile (300) avec le jeton d'autorisation enregistré (12) qui lui a été attribué par une entité autorisée (100, 200) habilitée à qualifier pour thérapie par l'utilisation de la composition pharmaceutique avec au moins un terminal qui génère un signal de commande (5) avec un schéma d'administration (11) et le jeton d'autorisation (12), et
en réponse à l'enregistrement du jeton d'autorisation (12) attribué au dispositif mobile (300) dans l'inhalateur (400), l'inhalateur est configuré pour traiter le schéma d'administration (11), en faisant du schéma d'administration (11) un schéma d'administration actif et en autorisant l'administration des doses d'une
composition pharmaceutique dans une fenêtre temporelle indiquée par le schéma d'administration (11), de sorte que l'inhalateur commande le moyen de blocage contrôlé (403) de manière à permettre l'administration de la composition pharmaceutique uniquement dans le respect du schéma d'administration (11), et en présence du dispositif mobile (300) avec le jeton d'autorisation enregistré (12) qui lui a été attribué par l'entité autorisée (100, 200).

2. L'inhalateur selon la revendication 1, dans lequel l'inhalateur est adapté pour être enregistré avec le jeton d'autorisation (12) attribué au dispositif mobile (300) en transférant le jeton d'autorisation (12) attribué au dispositif mobile du patient (300) dans la mémoire (404) de l'inhalateur (400).

3. L'inhalateur selon la revendication 1 ou 2, dans lequel
l'inhalateur (400) comprend en outre une unité de
détection (405) et est adapté pour
mesurer, via l'unité de détection (405), au moins une propriété physique du processus d'administration de la composition pharmaceutique sous forme de poudre sèche à l'intérieur de l'inhalateur (400) au cours d'un processus d'administration et
communiquer la propriété physique mesurée à l'appareil mobile (300).

4. L'inhalateur selon la revendication 3, dans lequel la propriété physique du processus d'administration de la composition pharmaceutique mesurée à l'intérieur de l'inhalateur (400) au cours d'un processus d'administration est une pression d'air, une intensité sonore, une magnitude de vibration ou toute combinaison de ces propriétés physiques.

5. L'inhalateur selon l'une des revendications 3 ou 4, dans lequel l'unité de détection (405) comprend un microphone, et la propriété physique mesurée est l'amplitude d'une onde sonore.

6. L'inhalateur selon l'une des revendications 3 à 5, dans lequel l'unité de détection (405) est placée à l'intérieur de la chambre de mélange où une composition pharmaceutique sous forme de poudre sèche est mélangée à l'air pendant l'inhalation.

7. L'inhalateur selon l'une des revendications 1 à 6, dans lequel le moyen de blocage contrôlé (403) comprend une unité d'entraînement et un élément d'actionnement actif qui bloque le transfert d'une dose de la composition pharmaceutique sous forme de poudre sèche depuis le moyen de stockage (410) vers le moyen d'administration (411).

8. L'inhalateur selon l'une des revendications 1 à 7 dans lequel le moyen de blocage (403) comprend un élément d'actionnement et l'élément d'actionnement dans un état de blocage bloque le transfert d'une dose de la composition pharmaceutique depuis le moyen de stockage (410) vers le moyen d'administration (411), et dans une position ouverte permet l'administration de la composition pharmaceutique en réponse à un signal de commande provenant du moyen de traitement (402).

9. L'inhalateur selon l'une des revendications 1 à 8, dans lequel le moyen de blocage (403) comprend un élément choisi dans un groupe comprenant : une valve, une goupille, un boulon, un relais, une clé, un interrupteur normalement fermé.
L'inhalateur selon l'une des revendications précédentes 1 à 9, dans lequel le moyen de stockage (410) comprend une composition pharmaceutique sous forme de poudre sèche comprenant de la kétamine ou son sel pharmaceutiquement acceptable pour une utilisation dans une méthode de traitement de la dépression, par administration directe aux poumons par voie pulmonaire

10. L'inhalateur selon l'une des revendications précédentes 1 à 9, dans lequel le moyen de stockage (410) comprend une composition pharmaceutique sous forme de poudre sèche comprenant de la kétamine ou son sel pharmaceutiquement acceptable pour une utilisation dans une méthode de traitement de la dépression, par administration directe aux poumons par voie pulmonaire.

11. L'inhalateur selon l'une des revendications précédentes 1 à 9, dans lequel le moyen de stockage (410) comprend de la kétamine ou son sel pharmaceutiquement acceptable pour une utilisation dans une méthode de traitement de la dépression, dans laquelle la kétamine ou son sel pharmaceutiquement acceptable est administré par voie pulmonaire sous la forme d'une composition pharmaceutique sous forme de poudre sèche.

12. L'inhalateur selon l'une des revendications 10 ou 11, dans lequel le sel pharmaceutiquement acceptable est du chlorhydrate.

13. L'inhalateur selon l'une des revendications 10 à 12, dans lequel la kétamine est du chlorhydrate d'eskétamine.

14. L'inhalateur selon l'une des revendications 10 à 13, dans lequel la composition comprend entre 2 mg et 100 mg de kétamine micronisée calculée en tant que base libre par dose unitaire nominale.

15. L'inhalateur selon la revendication 14, dans lequel la composition comprend entre 2 mg et 40 mg de kétamine micronisée calculée en tant que base libre par dose unitaire nominale.
